# EUROPEAN PATENT SPECIFICATION

(11) **EP 2 591 770 B1**
(45) Date of publication and mention of the grant of the patent: **16.03.2016**
(21) Application number: 12192681.0
(22) Date of filing: 14.11.2012
(51) Int. Cl.: A61K 9/51, C12N 15/113, A61K 9/20, A61P 35/00

(54) **Compositions for siRNA delivery and methods of manufacturing and using same**
Zusammensetzungen zur siRNA-Freisetzung und Verfahren zu ihrer Herstellung und Verwendung
Compositions pour administration de siRNA et procédés de fabrication et d'utilisation de celui-ci

(30) Priority: 14.11.2011 US 201161629136 P
(43) Date of publication of application: 15.05.2013
(73) Proprietor: Silenseed Ltd, 9112001 Ein Karem, Jerusalem (IL)
(72) Inventor: Shemi, Amotz, 4634826 Herzliya (IL); Malka Gabai, Rachel, 9987000 Mata (IL)
(74) Representative: Schulz Junghans Patentanwälte PartGmbB

(56) References cited:
- WO-A1-2009/006905
- WO-A2-2010/001325
- US-A1- 2009 258 924
- ALSHAMSAN AWS ET AL: "STAT3 Silencing in Dendritic Cells by siRNA Polyplexes Encapsulated in PLGA Nanoparticles for the Modulation of Anticancer Immune Response", MOLECULAR PHARMACEUTICS, AMERICAN CHEMICAL SOCIETY, US, vol. 7, no. 5, 1 January 2010 (2010-01-01) , pages 1643-1654, XP008160373, ISSN: 1543-8384, DOI: 10.1021/MP100067U [retrieved on 2010-08-30]
- GALUN EITHAN ET AL: "RNAI-BASED PANCREATIC CANCER THERAPY BY LOCAL DELIVERY OF ANTI K-RAS(MT) SIRNA", NUCLEIC ACID THERAPEUTICS, vol. 21, no. 5, October 2011 (2011-10), page A33, XP002708702, & 7TH ANNUAL MEETING OF THE OLIGONUCLEOTIDE-THERAPEUTICS-SOCIETY; COPENHAGEN, DENMARK; SEPTEMBER 08 -10, 2011
- MENGLIN CHEN ET AL: "Chitosan/siRNA Nanoparticles Encapsulated in PLGA Nanofibers for siRNA Delivery", ACS NANO, vol. 6, no. 6, 26 June 2012 (2012-06-26), pages 4835-4844, XP055054251, ISSN: 1936-0851, DOI: 10.1021/nn300106t

## Description

The present invention relates to polymeric implants containing microparticles or nanoparticles and a drug, and methods of delivery of microparticles and nanoparticles from a polymeric implant.

Delivery of drugs into solid tumors using clinically viable formulations remains a major technical hurdle. Significant effort has been devoted to exploring novel delivery strategies in drug delivery, specifically RNAi (RNA Interference) agents such as siRNA (small interfering RNA). Physiological constrains such as low permeability of the tumor vasculature and elevated interstitial pressure impose barriers to the efficiency of drug delivery. Low intratumoral diffusion rates can limit drug distribution to ~5-7 cells away from the blood vessel delivering the drug.

Tumors are considered to have four types of regions, based on their perfusion rates (Jain, Annu. Rev. Biomed. Eng. 1999; 1:241-63):
1. avascular necrotic region (blood flow rate: low, ~10% compared to collateral)
2. seminecrotic region (blood flow rate: low)
3. stabilized microcirculation region (blood flow rate: variable, can be high)
4. advancing front (blood flow rate: variable, can be high)

Particles in the bloodstream, even those that reach the tumor vasculature, remain at the better-vascularized regions (the aforementioned regions 3 and 4). However, as hypoxia is a major trigger for tumor progression, it is extremely important to reach internal regions (such as regions 1 and 2). With systemic administration, the amount of drug that reaches and remains within the tumor is low and distributed heterogeneously, requiring strategies to eliminate overdosing, toxicity and side effects; preserve the drug against degradation in the bloodstream; improve targeting; and improve penetration into the deepest tumor layers. Stable nucleic acid lipid particles (SNALP), although found recently to mediate more efficient siRNA delivery than any other delivery systems, still only deliver siRNA to 10-15% of a tumor, when delivered systemically (Wang et al., Gene Therapy (2012) 19, 775-780). Thus, a continuing need exists for compounds and methods of improving drug delivery to tumors.

Ramanujan et al. (Biophysical Journal (2002) 83:1650-1660) explored diffusion of particles in collagen gels, and found a log inverse ratio of diffusion D (cm²/sec) to the hydrodynamic radius of a particle.

Lieleg et al. (Biophysical Journal Volume 97 September 2009 1569-1577) showed that mobility of particles in the extracellular matrix depends on their zeta potential. Particles with zeta values of < -30mV or >10mV were immobile. The dependency was found to be non-linear and is best described as a 'cutoff' in mobility of nanoparticles in extracellular matrix at Zeta lower than about -30mv or higher than about 10-20mV.

WO2010/001325A2 discloses PLGA implants comprising RNAi agents complexed by polyethyleneimine (PEI).

WO2009/006905A1 shows chitosan nanoparticles comprising siRNA agents.

US2009/0258924A1 shows PLA or PLGA millimeter size implants containing siRNA. The RNA agent is formulated with PEG as excipient.

PLGA particles containing siRNA complexed with PEI are described, for example, by Alshamsan et al. 2010 (STAT3 Silencing in Dendritic Cells by siRNA Polyplexes Encapsulated in PLGA Nanoparticles for the Modulation of Anticancer Immune Response Molecular Pharmaceutics 2010, 7(5):1643-1654). Such particles generally exhibit relatively fast drug release, typically on the scale of one week, and are thus unsuitable *per se* for extended release.

Described herein are compositions containing small particles (the terms "small particles" and "particles" are used interchangeably herein) incorporated into a polymeric matrix, wherein the small particles themselves contain a polymeric material. The polymeric material in the particles is different from that in the matrix.

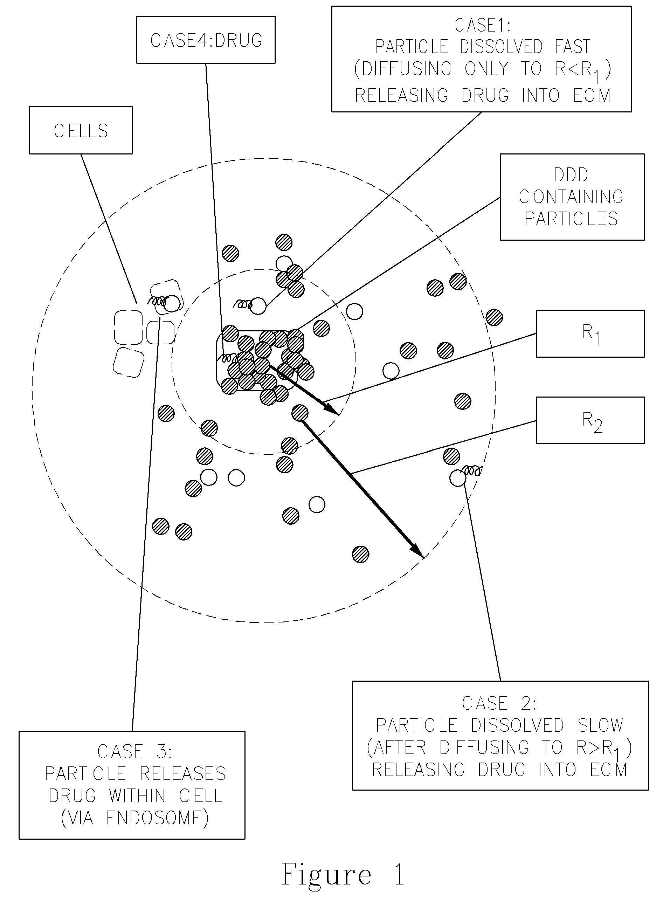

Also described are DDDs (drug delivery devices) comprising the compositions and methods of producing and using same. Implants containing polymeric matrices have been shown to be effective for extended delivery of drugs such as siRNA; however, the radius of effective drug release is suboptimal for certain therapeutic applications. The purpose of certain embodiments of the present disclosure is to increase the effective radius of drug release from such implants, as described herein with reference to Figure 1.

Mention herein of the particles as being "incorporated within" the matrix is not meant to imply that that the matrix must have a particle-free coating or "skin" around the region with the particles; rather the meaning is that the particles are dispersed within the matrix. Additionally, mention herein of the RNAi agent as being "incorporated within" the particles is not meant to imply that that the particles must have a RNAi agent-free coating or "skin" around the region with the RNAi agent; rather the meaning is that the RNAi agent is dispersed within the particles.

The effective radius is achieved by designing particles that are optimized by parameters affecting their diffusive mobility within the tumor tissue, and the amount of drug delivered by such particles to a defined distance from the implant. Design parameters include the particle radius, zeta potential, and degradation time, as well as the potential for high drug load, behavior in normal and acidic environments, and manufacturing parameters affecting the association of polymeric particles with a polymeric DDD. Studies of *in vitro* biodegradation via hydrolysis of PLGA showed that both alkaline and strongly acidic media accelerate polymer degradation (Holy et al., Biomaterials 1999,20, 1177-1185).

However, compositions of the present disclosure are able to achieve a lag period, where such particles are slowly released from the DDD, after which they diffuse within the surrounding tissue and drug release begins. In still other embodiments, there is an additional lag time after release from the DDD before drug release begins.

### Definitions

Unless specified otherwise, "drug" as used herein refers to an RNAi agent. In other embodiments, other drugs may be used, alternatively or in addition.

Nanoparticles - particles of size within the range 4 nm - 800 nm. (The lower size of ~4 nm typifies the smaller particles described here, which in typical embodiments is not a sphere, but a molecular complex, for example a drug molecule such as an siRNA molecule that is complexed with a polymer or conjugated to an additional molecule(s))

Studies of structural characteristics of complexes formed with siRNA show smaller lamellar spacing of a few nanometers (Desigaux et al., Proc. Nat. Acad. Sci. USA , 2007 vol. 104 no. 42, 16534-16539 and references therein). Peptide-conjugated siRNAs were able to form compact and stable nanosized (~6 nm) polyplex particles (Dohmen et al., Nanosized multifunctional polyplexes for receptor-mediated siRNA delivery. ACS Nano 2012 Jun 26;6(6):5198-208, and references therein). Here we consider 4 nm as the typical smallest dimension of the described particles.

Microparticles - particles having a size within the range 0.8 µm - 5 µm (also referred to as microspheres).

"Size" as used in this context refers to the largest dimension of a particle.

Small Particles (or 'particles') - nanoparticles and microparticles, including particles referred to as vesicles and/or spheres, in the size range or 4 nm - 5 µm.

### Brief description of the Figures

Figure 1: Schematic depiction of drug-release patterns of particle-containing DDD's. Several exemplary cases are presented, relating to the timing and location of drug release, as compared to the particle dissolving time and diffusion time. Examples include case 1: particles that dissolve rapidly (at radius R < R₁) compared to their diffusion time, where the drug is released R < R₁ and typically continue diffusing outwards; case 2: particles that dissolve at a slower rate (R₁ < R < R₂) compared to their diffusion rate, where the drug is mainly released from the particles during their transit; case 3: particles penetrating into cells, releasing the drug at that juncture, and case 4: drug molecules in DDD that are not incorporated in particles, diffusing out directly from the DDD.
Figure 2: Schematic depiction of the extension of effective radius of drug release (vertical axis). In this parameter set, the effective radius (horizontal axis) is increased from ~2mm to ~11mm, and the effective volume is increased by a factor of about 166.

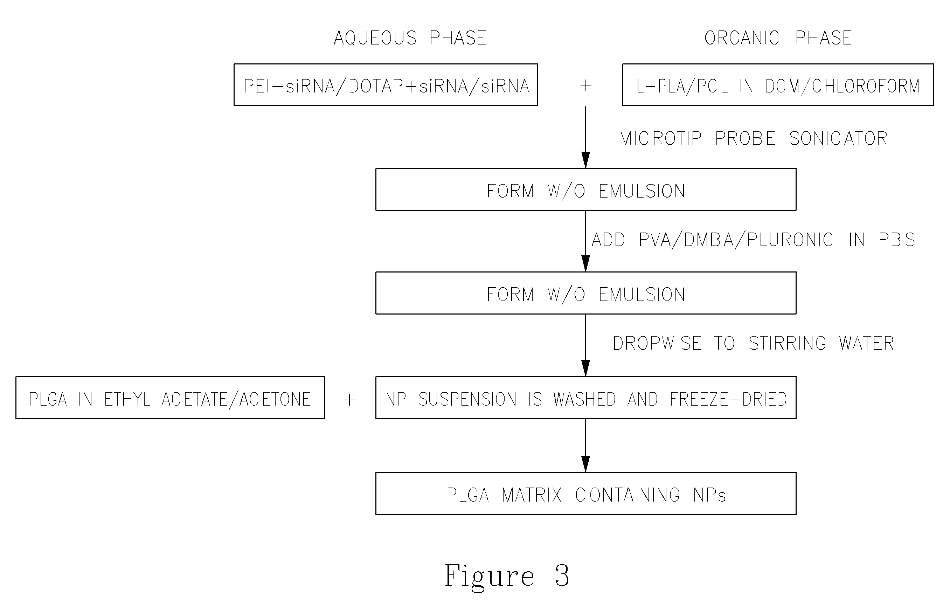

Figure 3: Flowchart of preparation of particle-containing DDD (Alshamsan A, Haddadi A, Incani V, Samuel J, Lavasanifar A, Uludag H. Formulation and delivery of siRNA by oleic acid and stearic acid modified polyethylenimine. Mol Pharm. 2009 Jan-Feb;6(1):121-33. PMID: 19053537.; Cun et al., High loading efficiency and sustained release of siRNA encapsulated in PLGA nanoparticles: quality by design optimization and characterization. Eur J Pharm Biopharm. 2011 Jan;77(1):26-35. Epub 2010 Nov 18. PMID: 21093589).
Figure 4: **A**-**B**. Zeta potential (**A**) and gel electrophoresis **(B)** of siRNA-DOTAP particles at various n/p ratios. **C.** Zeta potential of siRNA complexed to PEI of MW=2,500 at various n/p ratios. **D.** Zeta potential of siRNA complexed to PEI of MW=25,000 at various n/p ratios. **E.** Gel electrophoresis of siRNA complexed to PEI of MW=2,500 or 25,000 at various n/p ratios.
Figure 5: Size (**A**) and zeta potential **(B)** of particles prior to incorporation into matrix.
Figure 6: Size (**A**) and zeta potential **(B)** of particles released after a three-week incubation of the matrix in PBS.
Figure 7: Scanning (**A**) and transmission **(B)** electron microscope images of particles released after a three-week incubation of the matrix in PBS in 37°C.
Figure 8. Time course of particle release from matrix.
Figure 9. Diffusion of particles from the matrix in gelatin. **A**. Images at t=0 (upper left) and t=4 hours (upper right), 1 day (d) (center left), 4d (center right), 6d (bottom left), and 7d (bottom right). **B**-**D**. Plot of diffusion distance vs. elapsed time **(B)** and the square root of elapsed time **(C),** and as a function of gelatin concentration **(D).**
Figure 10. Diffusion of naked siRNA released from the matrix in gelatin.

### Sequence listing

The nucleic and/or amino acid sequences listed in the accompanying sequence listing are shown using standard letter abbreviations for nucleotide bases, and three letter code for amino acids, as defined in United States 37 C.F.R. 1.822. Only one strand of each nucleic acid sequence is shown, but the complementary strand is understood as included by any reference to the displayed strand. In the accompanying sequence listing:
SEQ ID NOs 1 and 2 are the sense and antisense strands of siG12D.
SEQ ID NOs 3-7 are exemplary sequences of cell-penetrating peptides.

Described herein are compositions containing small particles incorporated into a polymeric matrix, wherein the small particles themselves contain, in some embodiments, a polymeric material, and the compositions can contain the drug within the matrix in addition to the drug encapsulated in the particles. Also described are DDDs comprising the compositions and methods of producing and using same. The DDD may administered to (inserted, implanted, injected and/or attached to) the body. In certain embodiments, the diameter (or longest dimension, in the case of a non-spherical device) of the DDD is at least 100-fold greater than the mean particle diameter by a factor of 100, and the volume of the DDD is at least 1,000,000-fold greater than the mean particle volume. In still other embodiments, the diameter (or longest dimension, as appropriate) and volume are 200-fold and 8,000,000-fold greater, respectively, than the mean dimensions of the particles. In yet other embodiments, the diameter (or longest dimension, as appropriate) and volume of the DDD are 400-fold and 64,000,000-fold greater, respectively, than the mean dimensions of the particles. In additional embodiments, the diameter (or longest dimension, as appropriate) and volume are 1000-fold and 1,000,000,000-fold greater, respectively, than the mean dimensions of the particles. In certain embodiments, DDD matrixes and other DDD designs as described in US 2011/0195123 (Shemi) are utilized.

The present invention provides an implant according to claim 1, and methods for preparing said implant according to claim 14, the composition comprising: a) small particles; b) a matrix comprising a polymeric material; and c) an RNAi (RNA interference) agent, wherein the small particles are incorporated within the matrix, and some or all of the RNAi agent is incorporated within the small particles.

The small particles also comprise a polymeric material. In these embodiments, the polymeric material in the particles is referred to below as the "first polymeric material", while the polymeric material in the matrix is referred to as the "second polymeric material".

"Identical to" refers to polymers with the same building blocks, in the same ratio, and with the same MW. "Identical to" in this context does not preclude polymers that contain different materials other than the polymer building blocks). The polymer in the particles is non-identical to the polymer in the matrix.

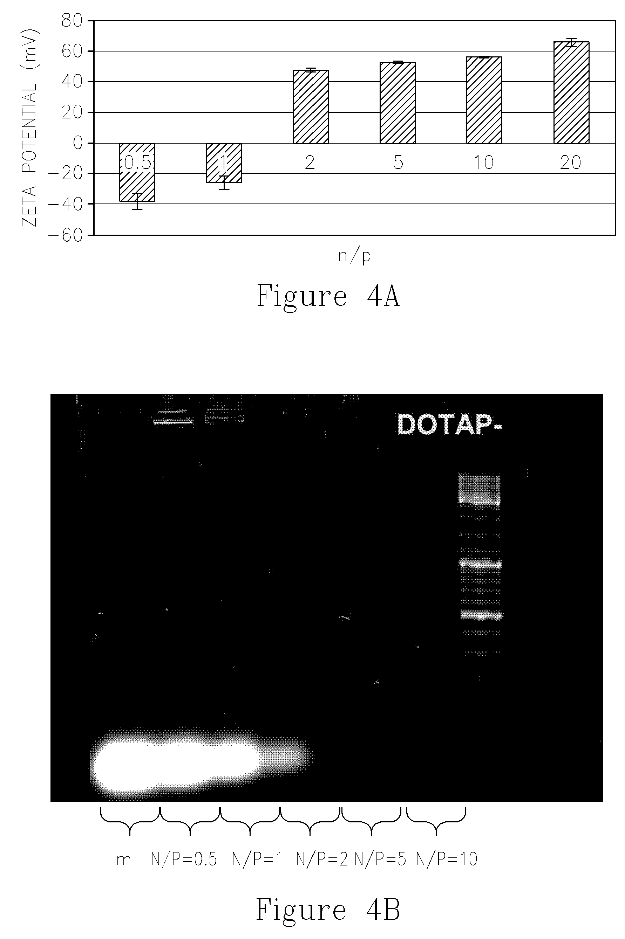

Also disclosed are small particles that do not comprise a polymeric-matrix. For example, the particles may be liposomes. Examples are particles comprising DOTAP or PEI, for example as presented in Figures 4A-4E, or another cationic molecule complexed with siRNA. Some examples appear in US 2011/0195123 (Shemi). As demonstrated herein, the electrostatic bonds between DOTAP and siRNA enable the complexed structure to be maintained when monomers of the matrix polymer and the complexes are suspended in, for example, an organic solvent, for example ethyl acetate, in turn enabling incorporation of such siRNA-containing complex particles into a matrix described herein.

In still another embodiment of siRNA-polymer complexes, small particles, for example siRNA-DOTAP complexes, are dissolved in chloroform and incorporated within larger PLA particles, and suspension of such PLA particles in ethyl acetate is mixed with PLGA to form a matrix.

In certain embodiments, all the RNAi agent in the composition is contained within the small particles. In other embodiments, there is also RNAi agent present within the matrix.

In other embodiments, the presence of drug both within and outside the particles enables biphasic drug release. The presence of drug outside the particles can achieve a higher drug load, for example as high as ~30% of the mass of the device. On the other hand, the presence of drugs within the particles enables a larger effective volume of drug penetration (Figure 1). The combination of these two phases thus simultaneously optimizes drug load and effective radius for certain applications.

In other embodiments, less than 50% by weight of the RNAi agent in the composition, for example less than 45%, 40%, 35%, 30%, or 25% of the RNAi agent is contained within the small particles. In still other embodiments, less than 20% by weight of the RNAi agent in the composition is contained within the small particles. In yet other embodiments, less than 18%, 16%, 14%, 12%, 10%, 8%, 7%, 6%, 5%, or 4% by weight of the RNAi agent in the composition is contained within the small particles.

In other embodiments the volume ratio [total volume of particles / volume of DDD] is less than 70%, less than 60%, less than 50%, less than 40%, less than 30%, or less than 20%, and the extra-particle volume contains additional drug.

### Materials

### Suitable biodegradable matrices

A variety of polymers may be used in the described methods and compositions. In some embodiments, one or more of the biodegradable matrices present in the composition comprises PLA. In other embodiments, the biodegradable matrix comprises poly(glycolic acid) (PGA). In other embodiments, the biodegradable matrix comprises PLGA. Methods for making PLGA matrices are known in the art. Methods of making PLGA matrices that incorporate RNAi agents are described inter alia in US Patent Application Pub. No. 2011/0195123 (Shemi). In other embodiments, any of Poly(D,L-lactide) (DL-PLA), poly(D,L-glycolide), or poly(D,L-lactide-co-glycolide) may be used, each of which is considered a separate embodiment. Exemplary methods of making biodegradable matrices are described in described in US Patent Application Pub. No. 2011/0195123, for example in Examples 1.1 and 1.2 thereof.

In other embodiments, the LA/GA ratio of PLGA used in the described compositions is between 25:75 and 75:25. In other embodiments, the ratio is between 50:50 and 75:25, meaning that there is between 50-75% LA and between 25-50% GA in the biodegradable matrix (discounting substances other than polymer building blocks). In other embodiments, the LA/GA ratio is between 25:75 and 50:50, between 35:65 and 75:25, between 45:55 and 75:25, between 55:45 and 75:25, between 65:35 and 75:25, between 75:25 and 35:65, between 75:25 and 45:55, between 75:25 and 55:45, or between 75:25 and 65:35.

In other embodiments, the LA/GA ratio is larger than 75:25, between 75:25 and 85:15, or between 75:25 and 95:5. Alternatively, the ratio is smaller than 25:75, between 25:75 and 15:85, or between 25:75 and 5:95.

In other embodiments, a polymer selected from the group consisting of PLGA, PLA PGA, and polyethylene glycol (PEG) is utilized in the composition. In other embodiments, the polymer comprises both PLA and PEG. In more specific embodiments, the polymer is a copolymer of PLA and PEG.

In other embodiments, tri-block PLA-PCL-PLA is used.

Design of biodegradable controlled drug-delivery carriers containing PLA, PGA, PEG, and/or PCL to have a specified release profile is well-within the ability of those skilled in the art, and is described *inter alia* in Makadia and Siegel, Poly Lactic-co-Glycolic Acid (PLGA) as Biodegradable Controlled Drug Delivery Carrier, Polymers 2011,3:1377-1397.

Polymers are described in paragraphs 0076-0078 of US Patent Application Pub. No. 2011/0195123.

As mentioned, the compositions typically have different polymeric materials within and outside the particles. In certain embodiments, the polymeric material within the particles is selected from the group consisting of DL-PLA (poly(DL-lactide)), L-PLA (poly(L-lactide)), PGA (poly(glycolide)), PCL (poly(ε-caprolactone), PLCL (Poly(lactide-co-ε-caprolactone). In more specific embodiments, the polymeric material within the particles is selected from the group consisting of L-PLA, PGA, PCL, and PLCL. PLCL may contain L-lactide, or DL-lactide. In more specific embodiments, the PLCL is DL-PLCL (Poly(DL-lactide-co-ε-caprolactone). In still more specific embodiments, the DL-PLCL is selected 25:75 DL-PLCP, or in another embodiment is 80:20 DL-PLCL. The ratio of lactide to ε-caprolactone may be any ratio, for example any of the ratios mentioned herein for PLGA. In yet other embodiments, the polymeric material within the particles is one of the above polymers, and the polymeric material in the matrix (outside the particles) is selected from the group consisting of DL-PLA and DL-PLGA). In yet other embodiments, the polymeric material within the particles is selected from the group consisting of L-PLA, PGA, PCL, PLCL, and the polymeric material in the matrix (outside the particles) is selected from the group consisting of DL-PLA and DL-PLGA).

In some embodiments, a polymer used in the described methods and compositions has a molecular weight (MW) of greater than 5 kilodaltons (kDa). In other embodiments, the MW is greater than 50 kDa. In other embodiments, the MW is greater than 7 kDa, greater than 10 kDa, greater than 15 kDa, greater than 20 kDa, greater than 30 Da, greater than 70 kDa, greater than 100 kDa, greater than 150 kDa, or greater than 200 kDa. In other embodiments, the MW is between 5-100 kDa, inclusive, between 7-80 kDa, inclusive, between 10-60 kDa, inclusive, between 20-50 kDa, inclusive, or between 25-50 kDa, inclusive.

In some embodiments, the polymer of the small particles is L-PLA that has a molecular weight of greater than 5 kilodaltons (kDa). In other embodiments, the MW of the L-PLA is greater than 50 kDa. In other embodiments, the MW is greater than 7 kDa, greater than 10 kDa, greater than 15 kDa, greater than 20 kDa, greater than 30 Da, greater than 70 kDa, greater than 100 kDa, greater than 150 kDa, or greater than 200 kDa. In other embodiments, the MW of the L-PLA is between 5-100 kDa, inclusive, between 7-80 kDa, inclusive, between 10-60 kDa, inclusive, between 20-50 kDa, inclusive, or between 25-50 kDa, inclusive.

In some embodiments, the polymer of the matrix is PLGA that has a molecular weight of greater than 5 kilodaltons (kDa). In other embodiments, the MW of the PLGA is greater than 50 kDa. In other embodiments, the MW is greater than 7 kDa, greater than 10 kDa, greater than 15 kDa, greater than 20 kDa, greater than 30 Da, greater than 70 kDa, greater than 100 kDa, greater than 150 kDa, or greater than 200 kDa. In other embodiments, the MW of the PLGA is between 5-100 kDa, inclusive, between 7-80 kDa, inclusive, between 10-60 kDa, inclusive, between 20-50 kDa, inclusive, or between 25-50 kDa, inclusive.

In some embodiments, the polymer of the matrix is PLGA that has a PLA:PGA ratio of between 80:20 and 90:10, inclusive, for example 80:20, 82:18, 84:16, 86:14, 88:12, or 90:10, and a MW of greater than 50KDa, for example greater than 50 kDa, greater than 70 kDa, greater than 100 kDa, greater than 150 kDa, or greater than 200 kDa. In other embodiments, the polymer has a PLA:PGA ratio larger than 75:25, for example 76:24, 78:22, 80:20, 82:18, 84:16, 86:14, 88:12, 90:10, 92:8, 94:6, 96:4, or 98:2, and a MW of greater than 50KDa. In yet other embodiments, the polymer has a PLA:PGA ratio smaller than 25:75, inclusive, for example 24:76, 22:78, 20:80, 18:82, 16:84, or 14:86, 12:88, 10:90, 8:92, 6:94, 4:96, or 2:98, and a MW of greater than 50KDa. Each of the aforementioned PLA:PGA ratios may be freely combined with each MW value.

In certain embodiments, all the polymeric materials utilized in the DDD are biodegradable materials.

### Particle characteristics

The small particles present in the matrix or DDD are of micrometer and nanometer scale. The size of the particles is between 4 nm - 5 µm inclusive, in other embodiments, between 25 nm - 5 µm inclusive, between 35 nm - 4.5 µm inclusive, between 50 nm - 4 µm inclusive, between 50 nm - 3 µm inclusive, between 50 nm - 2 µm inclusive, between 50 nm - 1 µm inclusive, between 50-500 nm inclusive, between 50-300 nm inclusive, between 50-200 nm inclusive, between 60 nm - 5 µm inclusive, between 60 nm - 4 µm inclusive, between 60 nm - 3 µm inclusive, between 60 nm - 2 µm inclusive, between 60 nm - 1 µm inclusive, between 60-500 nm inclusive, between 60-300 nm inclusive, or between 70-200 nm inclusive.

Also disclosed are PLGA matrix particles, liposomes, for example stable nucleic acid lipid particles (SNALP), bilayer and multilayer liposomes, LNPs (lipid nanoparticles), Lipoplexes for example as described in Metwally et al, particles comprising epoxide-derived lipidoid materials such as C12-200, as described in Love et al. (Proc Natl Acad Sci USA (2010), 107: 1864-1869), polymersomes, such as those including TCOsomes derived from tocopherol and chitosan, and complexes of RNAi-based drugs with polymers. Other examples of liposomes are so-called "smart" stimuli-sensitive liposomes that respond to certain internal or external stimuli, such as pH, temperature, redox potential or magnetic field. Any type of liposome known in the art may be utilized.

SNALP Liposomes can either contain or lack PEG. They can be prepared for example, by formulating D-Lin-DMA and PEG-C-DMA with distearoylphosphatidylcholine (DSPC), cholesterol and siRNA. Wang et al. (Gene Therapy (2012) 19, 775-780) describes a 25:1 lipid/siRNA ratio and a 48/40/10/2 molar ratio of Cholesterol:D-Lin-DMA:DSPC:PEG-C-DMA; however, those skilled in the art will appreciate that a variety of suitable ratios may be utilized. In some embodiments, the SNALP liposomes that are used are 80-100 nm in size, with a PDI < 0.1 and a zeta potential within 10 mV of zero (-10 mV to +10 mV).

The compositions described herein are able to achieve a lag period, wherein particles are slowly released from the DDD, after which they diffuse within the surrounding tissue and drug release begins. In still other embodiments, there is an additional lag time after release from the DDD before drug release begins. In some embodiments, the particles are composed of PLA, PLA-PEG, or PLGA with a PLA:PGA ratio larger than 75:25 or smaller than 25:75.

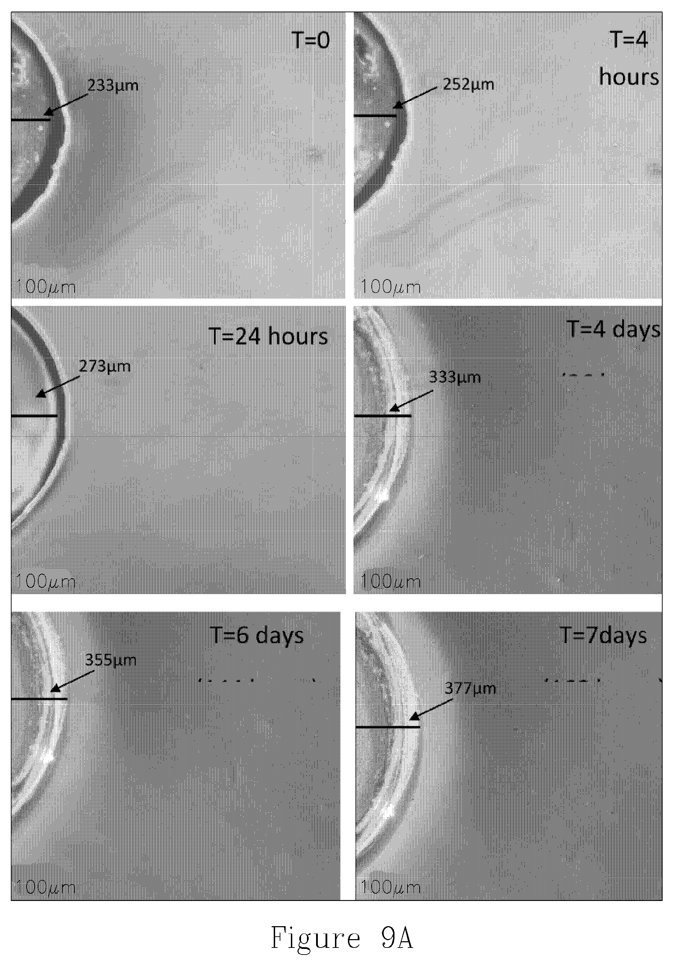

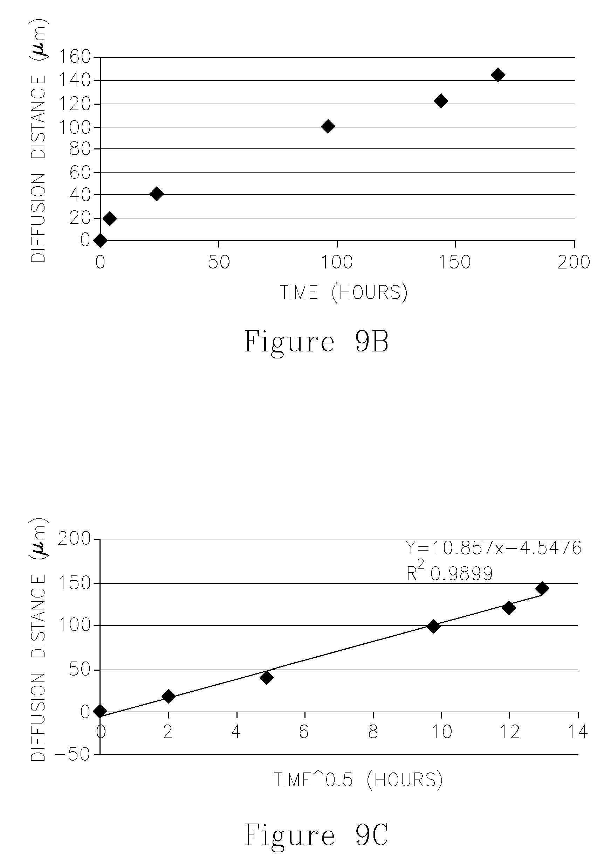

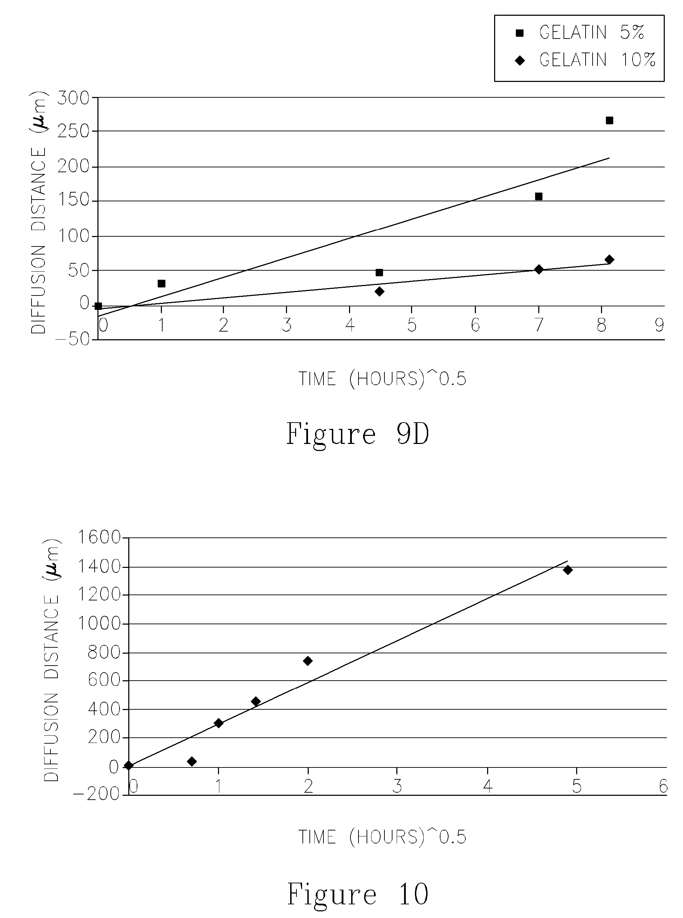

As provided herein, release of particles from 6-coumarin-containing matrices described herein into 10% gelatin and 5% gelatin was studied (Figure 9A-D). Diffusion in gelatin is well known in the art as a laboratory model for testing mobility of molecules and particles in medium that simulates the extracellular matrix (ECM). Diffusion coefficients in the ECM can vary from ~10⁻⁵ cm²/sec (rapid diffusion) to ~5*10⁻¹⁰ cm²/sec (extremely slow diffusion), where in general small molecules, or particles of smaller diameter and/or zeta potential closer to neutral values, diffuse faster. The diffusion distance is proportional to the square root of the elapsed time (Figure 9B-D) and is best fitted in the example of Figure B-C to 3.36* 10⁻¹⁰ cm²/sec. In this example, the very high gelatin concentration (10%) was selected to simulate very dense ECM conditions, where the diffusion is extremely slow. Showing here is a DDD of radius (at t=0) 233 µm, and the effective radius (DDD + diffused particles) after 7days is 377 µm. The effective volume therefore increased more than fourfold in seven days [(377/233)³ = 4.236]. In general, naked siRNA would diffuse significantly faster than nanoparticles. For example, the diffusion coefficient in Figure 10 is best fitted to 2.4*10⁻⁷ cm²/sec. However, after a week most siRNA molecules in the ECM would likely be degraded. Therefore, extending the effective volume of siRNA release is desired.

Faster diffusion is presented in Figure 9D, where the gelatin concentration is 5%, and D = 2.18*10⁻⁹ cm²/sec. These values are closer, but still lower than, the range of diffusion coefficient of macromolecules and particles measured in tumor models (Brown et al., Microvascular Research 67 (2004) 231- 236).

This shows that particles diffuse at a significant rate from an implanted polymeric matrix into surrounding tissue and thus have the potential to increase the range of drug release from an implanted polymeric matrix.

In other embodiments, the small particles present in the matrix exhibit degradation in an aqueous environment in a pH-sensitive fashion. In some embodiments, low and high pH accelerate polymer degradation (Gopferich, Biomaterials. 1996;17 (2):103-14; Holy, *ibid*.). More specifically, the particles may be more stable in an aqueous solution having a pH of < 5.5 at 37°C, compared to an aqueous solution having a pH of > 6.5 at 37°C. In one embodiment the particles are stable in an aqueous solution having a pH of < 5.5 for a period of at least 1 month, for example 1 month, 2 months, 3 months, 4 months, 6 months, 8 months, 10 months, 12 months, 15 months, 18 months, 21 months, or 24 months, and undergo degradation in an aqueous solution having a pH of > 6.5 within 1-8 weeks, for example 1 week, 2 weeks, 3 weeks, 4 weeks, 5 weeks, 6 weeks, 7 weeks, or 8 weeks. Each of the aforementioned time frames for stability and degradation may be freely combined. As used herein, "stable" indicates a ratio of the Molecular Weight (MW) at the end of the period to the MW at time zero that is larger than 80%. Alternatively, for example in the case of a homogeneous particle, "stable" may refer to a decrease of the particle radius of 10% or less during the measuring period. In other embodiments, supporting data such as porosity of the surface as imaged by for example SEM, AFM or TEM can be provided.

In other embodiments, the half-life of the drug within the particles (the time at which 50% of the drug is released) at a pH of 5.0 at 37°C is at least 5 times, in other embodiments at least 6 times, at least 8 times, at least 10 times, at least 12 times, at least 15 times, at least 20 times, at least 30 times, at least 50 times, or at least 100 times greater than the half-life of the drug within the particles at a pH of 7.0 at 37°C.

In still other embodiments, the half-life of the drug within the particles (the time at which 50% of the drug is released) at a pH of 4.0 at 37°C is at least 5 times, in other embodiments at least 6 times, at least 8 times, at least 10 times, at least 12 times, at least 15 times, at least 20 times, at least 30 times, at least 50 times, or at least 100 times greater than the half-life of the drug within the particles at a pH of 6.0 at 37°C.

The inventors have discovered that conditions inside a DDD can become acidic, within the pH range of 2.5-6.5. In tumor tissue, by contrast, the pH is often between 6.5-7.2. Thus, the aforementioned particles will be stable prior to release from the matrix, but will degrade relatively rapidly after release in a subject in the vicinity of tumor.

Polymers relatively stable at low pH include, inter alia, PLA, Polycaprolactone (PCL), and polyanhydrides. Polyanhydrides can be prepared by the reaction of a dicarboxylic acid with an excess of acetic anhydride or acetyl chloride. They include aliphatic anhydride polymers, unsaturated anhydride polymers, for example fumaric acid, and aromatic anhydride polymers and copolymers, and have the structure shown below as Formula I. In Formula I, the R group is saturated carbon chain, typically having 3-16 carbons, that is unsubstituted or, in other embodiments substituted, for example with a nitro, amino, hydroxyl, or halogen moiety.

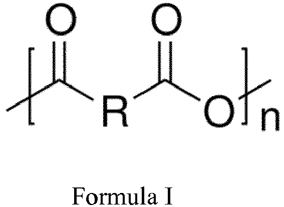

Aliphatic anhydride polymers include polymers and copolymers, for example block copolymers composed of, or in other embodiments comprising, sebacic acid, adipic acid, suberic acid, dodecanedioic acid, alpha-ketoglutaric acid, succinic acid, oxaloacetic acid, malonic acid, glutaric acid, pimelic acid, or azelaic acid.

In still other embodiments, the particles are stable at low pH (as described above), while the matrix surrounding the particles is degraded in a pH-insensitive fashion between pH 3.0-7.0 at 37°C. In certain embodiments, the matrix is made of a polymer that at which the rate of degradation does not differ more than 5-fold, in other embodiments not more than 6-fold, not more than 8-fold, not more than 10-fold, not more than 15-fold, or not more than 20-fold, between pH 5.0 and 7.0, or in other embodiments, between pH 4.0 and 6.7.

In still other embodiments, the particles remain stable within the DDD *in vivo* until they diffuse out to the surrounding tissue (including the ECM). In still other embodiments, the particles remain stable within the DDD *in vivo* for at least one week. In other embodiments, the period of stability within the DDD *in vivo* is at least two weeks. In yet other embodiments, the aforementioned period of stability is at least four weeks. In other embodiments, the period of stability is at least two months. In other embodiments, the period of stability is at least three months. In other embodiments, the period of stability is at least four months.

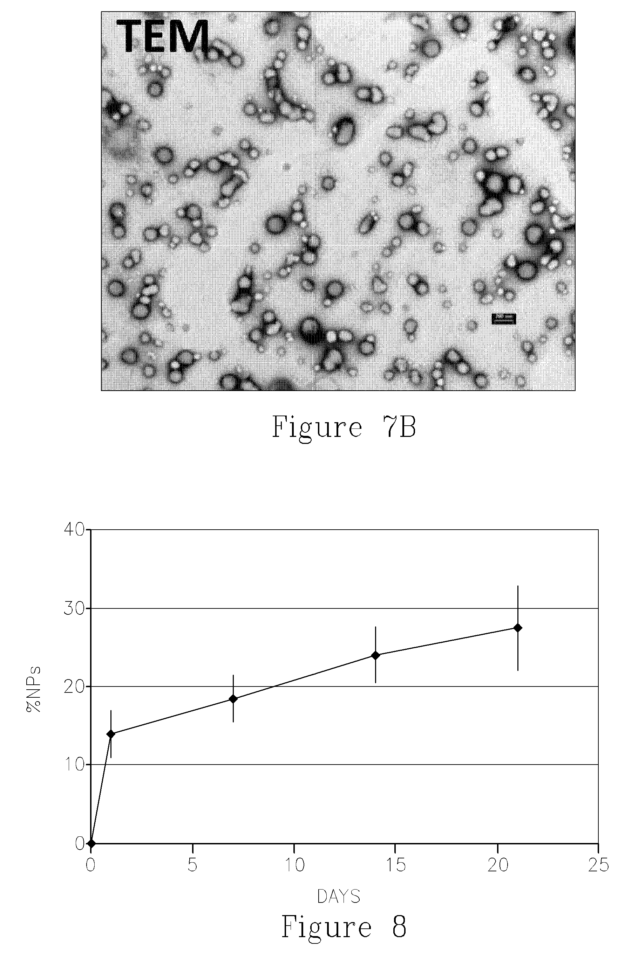

In other embodiments, the particles from the matrix are released at an essentially constant rate (in some embodiments after a short period of faster release, for example as shown in Figure 8), starting at least 24 hours from the time the DDD is administered to the body. As used herein an "essentially constant" rate refers to "zero-order" type of release curves, where the rate, in terms of drug per day, is essentially constant throughout most of the release period. The release period in this context could be, in various embodiments, 3 days, 1 week, 2 weeks, 3 weeks, 4 weeks, 2 months, 3 months, 4 months, 5 months, 6 months, 8 months, 10 months, 12 months, 15 months, 17 months, 20 months, or 24 months.

In other embodiments, the small particles in the device or matrix have a single major size peak mean in the range between 20 to 500 nm, inclusive. A distribution is considered to have a single major size peak if there is only 1 peak that contains 20% or more of the total particles. In other embodiments, there is a single major size peak, and the mean size within that peak is between 20 to 500 nm, inclusive.

In other embodiments, the small particles in the device or matrix have at least a biphasic size distribution, or alternately a biphasic size distribution or a distribution with at least 2 peaks. In some embodiments, when the size distribution of the particles is plotted, there is a first size peak larger than 200 nm, and a second size (or size) peak (or additional peak, if more than 2 peaks are present) smaller than 120 nm. In other embodiments, there are 2 major size peaks, one of which has a mean size larger than 200 nm, and the second of which has a mean size smaller than 120 nm. In other embodiments, the first size peak is between 250-1000 nm inclusive, for example 200 nm, 250 nm, 300 nm, 400 nm, 500 nm, 750 nm, or 1000 nm, and the second (or additional) size peak is between 4-120 nm inclusive, for example 4 nm, 10nm, 18 nm, 20 nm, 30 nm, 50 nm, 70 nm, 90 nm, or 120 nm. In still other embodiments, second (or additional) size peak is smaller than 80 nm, or in other embodiments between 10-80 nm inclusive, for example 10 nm, 15 nm, 20 nm, 30 nm, 40 nm, 50 nm, 60 nm, 70 nm, or 80 nm. In still other embodiments, second (or additional) size peak is smaller than 40 nm, or in other embodiments between 10-40 nm inclusive, for example 10 nm, 15 nm, 20 nm, 30 nm, or 40 nm. Size peaks in this context are considered significant if they contain at least 20% of the total particles. Each embodiment of the aforementioned large and small particle sizes may be freely combined.

In some embodiments, the larger particles avoid uptake into cells, thus (although their diffusion is slower than smaller particles under optimized conditions) they diffuse further from the implanted DDD, while the smaller particles are taken up by phagocytosis, thus acting on tumor cells in the immediate vicinity of the DDD. In other embodiments, particles typically smaller than 40 nm facilitate endosome-mediated cell uptake.

As provided herein, polymeric matrices containing particles can be formed wherein the particles maintain their structure integrity when the matrix is incubated under physiologic conditions for one week, or in other embodiments two weeks, one month, two months, three months, six months, or longer. In other embodiments, the particles substantially maintain their structural integrity when the matrix is incubated in PBS at 37°C for one week, or in other embodiments two weeks, one month, two months, three months, six months, or longer. In other embodiments, the particles substantially maintain their structural integrity when the matrix is incubated under storage conditions, for example between -20°C - +55°C, for one week, or in other embodiments two weeks, one month, two months, three months, six months, or longer. The structural integrity of particles can be measured by releasing the particles from the matrix, for example by dissolving the matrix, and performing microscopy or measuring the size distribution or zeta potential of the particles. "Substantially maintain their structural integrity" in this context means that none or less than 20% of the particles exhibit statistically significant structural degradation as measured by microscopy, or alternatively by size measurement or zeta potential.

In certain embodiments, the particles, once released from the DDD *in vivo,* travel within the ECM (extracellular matrix), for example by diffusion and/or convection. In some embodiments, the particles have a relatively short half-life of less than 30 days, for example less than 2 days, less than 3 days, less than 5 days, less than 7 days, less than 10 days, less than 15 days, less than 20 days, or less than 30 days. "Half-life" in this context refers to the time at which the particles are no longer detectable by visualization it the ECM. The relatively short half-life may be due to the particles comprising a rapidly-degrading polymer, thus releasing drug into the ECM, or their being of a size amenable to penetrating and/or being engulfed by the cell membrane, including by phagocytosis, thus releasing drug directly into the cells, following endosomal disruption.

In other embodiments, the particles have a longer half-life in the ECM of more than 30 days, for example more than 30 days, more than 45 days, more than 60 days, more than 90 days, more than 120 days, more than 150 days, more than 200 days, or more than 300 days. The relatively long half-life may be due to the particles comprising a more slowly-degrading polymer and being of a size too large amenable for phagocytosis. Once the particle begins to degrade, it releases its drug as a direct result of hydrolysis and/or degradation and/or after reaching a size amenable to phagocytosis.

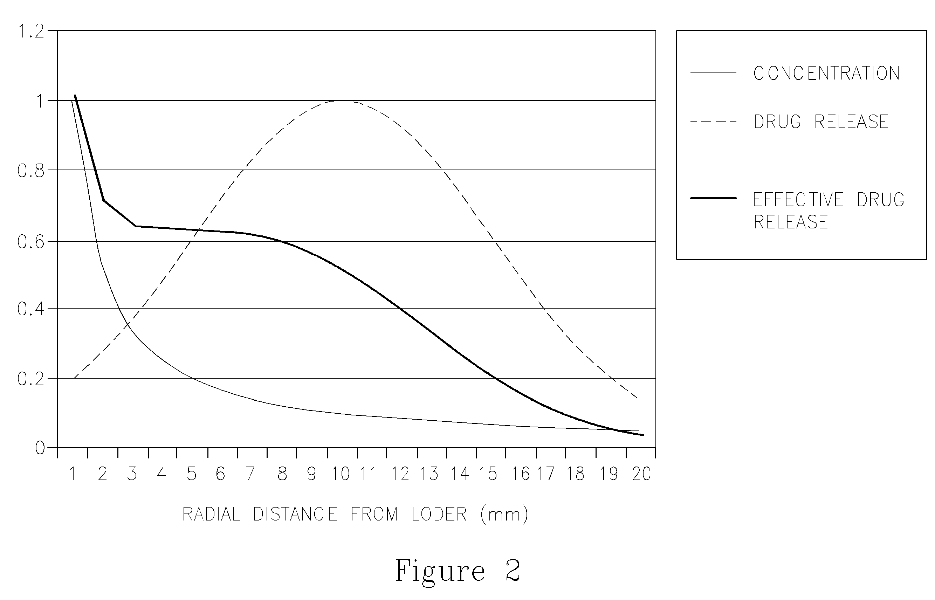

For example, particles made from PLA (including the PLA particles described in Lassalle and Ferreira, Macromol. Biosci. 2007, 7:767-783), PLA-PEG, PLGA (of all PLA:PGA ratios) PLA-PEI, or chitosan can remain essentially stable within the DDD, until they diffuse out of the DDD. As illustrated for example in Figures 1-2, particles then diffuse within the ECM away from the DDD as a function of time r(t) ≈ R₍₀₎ x [D_{Diffusion} x t]^{1/2} (convection of particles is ignored here). Particles dissolve at a typical time and typical distribution width (for example a typical time τ₁ typical distribution width τ₂ dissolving time ~ exp [-(t-τ₂)/ τ₁ ]^2). The effective radius (and thereby volume) will be the convolution of such two time-dependent functions. Along this radius, the particles can disintegrate, e.g. by cell uptake and/or degradation. If the time for cell uptake is longer than chemical degradation, the drug will be released into to the ECM, then be taken up into cells. Therefore, in some embodiments, particles that are anionic, lipophobic, and/or large (for example > 1 micron in diameter) are used. Provided herein are particles that are optimized, in some embodiments, in their radius, zeta, drug load and degradation time.

In some embodiments, the DDD is characterized by the fraction of stable particles, among those particles remaining inside the DDD, after implantation in the human body. In one embodiment, at least 90% of the particles remain stable for at least 1 week. In another embodiment, at least 90% of the particles remain stable for at least 2 weeks. In another embodiment, at least 90% of the particles remain stable for at least 4 weeks. In another embodiment, at least 75% of the particles remain stable for at least 1 week. In another embodiment, at least 75% of the particles remain stable for at least 2 weeks. In another embodiment, at least 75% of the particles remain stable for at least 4 weeks.

In some embodiments, the DDD is characterized by the amount of degradation of particles within the DDD under the conditions specified in the preceding paragraph, while at the same time the particles are relatively unstable inside the human body after release from the DDD. In one embodiment, at least 10% of the released particles are degraded after 1 week. In another embodiment, at least 25% of the released particles are degraded after 1 week. In another embodiment, at least 50% of the released particles are degraded after 1 week. In another embodiment, at least 10% of the released particles are degraded after 2 weeks. In another embodiment, at least 25% of the released particles are degraded after 2 weeks. In another embodiment, at least 50% of the released particles are degraded after 2 weeks. In another embodiment, at least 10% of the released particles are degraded after 4 weeks. In another embodiment, at least 25% of the released particles are degraded after 4 weeks. In another embodiment, at least 50% of the released particles are degraded after 4 weeks. In other embodiments, the stability values following release may be simulated in an aqueous medium, at a temperature of 37°C-39°C and a pH of 6.5 to 7.5.

In some embodiments the zeta potential of the particles as measured at pH = 6.75 is negative, which in general slows cellular uptake. In other embodiments, the zeta potential is essentially neutral, namely 0.0 +/- 2.0mV, which enables faster diffusion in the ECM. In other embodiments, the particles are of positive zeta potential as measured at pH = 6.75. Zeta potential values may range between -45 to +45 mV inclusive, in other embodiments between -35 to +35 mV inclusive, between -30 to +25 mV inclusive, between -30 to +20 mV inclusive, between -30 to +15 mV inclusive, between -25 to +10 mV inclusive, or between -15 to +8 mV inclusive. In this context, the zeta potential referred to is the potential of the particles prior to incorporation into the matrix. Alternatively, the zeta potential may be measured following incubation of the matrix in PBS at 37°C, followed by dissolution of the matrix in a suitable solvent, or example ethyl acetate.

The dispersion of the particles within the matrix, in one embodiment, is homogenous. In other embodiments, the dispersion is non-homogenous. For example, a non-homogenous particles dispersion occurs when particles are confined to an internal region (or a semi-internal region such as the middle part of a cylinder) within the matrix. In other embodiments, the particles are concentrated within the inner volume of the DDD that is less than 3/4 of the total volume of the DDD. In still other embodiments, a non-homogenous dispersion is achieved by coating a DDD, for example coating with a polymer selected from PLA, PLGA 85:15, and PLGA 50:50. In certain embodiments, while mannitol is present in the particles, there is no mannitol in the coating. Coating can be achieved by methods known in the art of dipping and/or spraying. In other embodiments, the DDD polymer containing particles is injected into a tube, and following a drying period, one or more additional layers of polymers without particles (or, in other embodiments, with a smaller particle concentration) are injected to the tube, preferably from both sides. In another method the DDD is manufactured by film(s), for example as described in US 2011/0195123 (Shemi). Extending this method, in addition to a film containing particles, more film layers are added, preferably at the bottom and upper sides, where these bottom and upper layers are made by polymer without (or with a smaller concentration of) particles.

In some embodiments, surface modification is applied to the particles for improving their stability, as described for example in Motoyuki and Kamiya (Surface Modification for Improving the Stability of Nanoparticles in Liquid Media. KONA Powder and Particle Journal No.27 (2009)).

In yet other embodiments, the particles are themselves a therapeutic agent; in other words, there are no molecules of a drug in the classical sense that are encapsulated in the particles. Examples are polymer particles designed to trigger an immune response. In these embodiments, there are not, or in other embodiments are, drug molecules encapsulated in the matrix.

In other embodiments, the particles consist of drug molecules, for example glucagon particles, such as those of Particle Therapeutics LTD, Yarnton UK).

### Additives

In other embodiments, the biodegradable matrix further comprises an additive for modulating hydrophilic-hydrophobic interactions; in other embodiments for enabling dispersion of the drug and eliminating aggregation; in other embodiments for preserving the drug in hot-temperature or cold-temperature storage conditions, for example 55°C and -20°C, respectively, or significantly colder, in the case of lyophilization with liquid nitrogen; in other embodiments for facilitating creation of cavities in the implant that affect to drug diffusion from the matrix, in other embodiments for modulating the decrease in pH in internal parts of the matrix. Hydrophilic-hydrophobic interactions may cause aggregation of the active substance in cases of hydrophilic active substances, such as siRNA, incorporated within a hydrophobic polymer, resulting in aggregation during production or subsequently when the device is implanted into the body of a subject and it is subjected for example to hydrolysis. Non-limiting examples of such additives are open monosaccharides, for example mannitol; disaccharides such as trehalose; sorbitol; and other cyclic monosaccharides such as glucose, fructose, galactose and disaccharides such as sucrose. The above additives, if chiral, may be in the form of the D-enantiomer, the L-enantiomer, or a racemic mixture. In other embodiments, more than one additive is present. Trehalose is of particular interest.

In other embodiments, the biodegradable matrix further comprises an additive for protecting the drug against low pH after implantation. The microenvironment in the implant interior tends to be acidic. Unlike chemotherapy, the pH should preferably be maintained above a threshold. While doxorubicin is stable in an acidic environment, with minimal hydrolytic degradation within a pH range of 3 to 6.5, RNAi drugs might degrade at pH <3. In more specific embodiments, this additive may be selected from bicarbonate and carbonate, for example sodium bicarbonate, sodium carbonate or magnesium hydroxide (Mg(OH)₂). In still more specific embodiments, a composition or DDD may contain 12-20% particles, 2-10% mannitol, 0.1-0.3% sodium bicarbonate, and 70-80% additional polymer. In yet more specific embodiments, the particles are composed of L-PLA, and the additional polymer is PLGA.

In some embodiments an additional substance, for example a cationic molecule, for example polyethylenimine (PEI), 1,2-dioleoyl-sn-glycero-3-phosphatidylethanolamine (DOPE), (N-[1-(2,3-Dioleoyloxy)propyl]-N,N,N-trimethylammonium) (DOTAP), 1,2-dioleoyl-sn-glycero-3-phosphocholine (DOPC), 1,2-di-O-octadecenyl-3-trimethylammonium propane (DOTMA), Spermine, or a PEI-PLA polymer is included in the matrix, in another embodiment in the particles, in another embodiment in both the matrix and the particles. The inclusion of cationic polymer in many cases raises the zeta potential and/or alters the size of the particles, the drug encapsulation ratio (or entrapment ratio), or the ratio of [drug entrapped in particles]/[total drug present]). Preferably, the ratio of cationic-polymer to other polymer building blocks is greater than 0.001%. In certain embodiments, DOTAP, complexed with, or in other embodiments not complexed with an siRNA, is itself the polymer that forms the structure of the particles.

In other embodiments, CS (chitosan), PEG (polyethylene glycol), PVA (polyvinyl alcohol), Zn²⁺, a fatty acid, or iron oxide is included in the polymer in the matrix.

In other embodiments, a DDD of the present disclosure is coated. A coating can be designed for a number of characteristics, including modulating the release rate or preventing protein stickiness during long-term storage. The coatings in some embodiments comprise the same material or a similar material used to form the matrix, for example a drug-loaded PLGA matrix, only without the drug, and in other embodiments comprise other materials such as PEG. In other embodiments, the coating selected from polylactic acid (PLA) and PLA:PGA (polyglycolic acid) in a ratio of at least 90:10, for example 90:10, 92:8, 94:6, 96:4, 98:2, and 99:1, and a MW greater than 50KDa, for example 60KDa, 70KDa, 80KDa, 100KDa, 120KDa, 1500KDa, or 200KDa).

In certain embodiments, DDD's of dimensions larger than ~0.8 mm can be identified by standard ultrasound (US). In other embodiments, materials to enhance visualization in a medical or surgical procedure, for example CT, MRI or US visualization are included. Non-limiting examples of contrast agents are barium, diatrizoate, ethiodized oil, gallium citrate, iocarmic acid, iocetamic acid, iodamide, iodipamide, iodoxamic acid, iogulamide, iohexyl, iopamidol, iopanoic acid, ioprocemic acid, iosefamic acid, ioseric acid, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, and thallous chloride.

In other embodiments, a surfactant such as PVP, gelatins, methyl cellulose, lecithin, or HSA is included in the matrix.

### RNAi agents

It will be appreciated by those skilled in the art in light of the present disclosure that a variety of RNAi agents may be used in the described methods and compositions. An RNAi agent is any RNA polynucleotide, or suitable analogue that can be utilized by the cellular RNA silencing machinery. One non-limiting example is following siRNA: GUUGGAGCUGAUGGCGUAGdTdT (sense strand; SEQ ID No: 1), and CUACGCCAUCAGCUCCAACdTdT (antisense strand; SEQ ID No: 2).

The term "nucleotide" as used herein refers to RNA nucleotides cytosine, guanine, adenine, and thymidine and to other similar nucleotide analogues suitable for use in RNAi agents. Such nucleotide analogues may generally be used as the building blocks of the RNAi agents described herein, except where indicated otherwise. Except where stated explicitly, nothing in this application is intended to preclude the utilization of non-classical nucleotide analogues, for example nucleotide analogues whose base or backbone has been chemically modified to enhance its stability or usefulness as an a RNAi agent, provided that they pair with the appropriate complementary bases, as previously mentioned. In certain embodiments, nucleotide analogues that may be used in the described methods and compositions include derivatives wherein the sugar is modified, as in 2'-O-methyl, 2'-O-allyl, 2'-deoxy-2'-fluoro, and 2',3'-dideoxynucleoside derivatives; 2'-O-benzyl, 2'-O-methyl-4-pyridine (2'-O-CH₂P_{y}(4)); nucleic acid analogs based on other sugar backbones, such as threose; locked nucleic acid derivatives; bicyclo sugars; hexose, glycerol and glycol sugars; nucleic acid analogs based on non-ionic backbones, such as "peptide nucleic acids", these nucleic acids and their analogs in non-linear topologies, including as dendrimers, comb-structures, and nanostructures, and these nucleic acids and their analogs carrying tags (e.g., fluorescent, functionalized, or binding) to the ends, sugars, or nucleobases.

In other embodiments, an RNAi agent used in the described methods and compositions is conjugated to a cholesterol moiety, a bile acid, a long-chain fatty acid or, in other embodiments, another lipid moiety.

In still other embodiments, an RNAi agent used in the described methods and compositions is conjugated to an α-tocopherol moiety.

### 2'-OMe Modifications

In more specific embodiments, an RNAi agent used in the described methods and compositions is chemically modified with a modification selected from 2'-OMe and 2'-O-methoxy on at least one residue of at least one strand thereof. In other, more specific embodiments, the RNAi agent is chemically modified with 2'-OMe or 2'-O-methoxy on at least one residue of each of its 2 strands. In still more specific embodiments, the modification is 2'-OMe. In yet more specific embodiments, the 2'-OMe modification is present on at least one residue of each of its 2 strands.

In even more specific embodiments, four 2'-OMe modifications are present on each strand of the RNAi agent. Alternatively, four 2'-OMe modifications are present on one strand of the RNAi agent, while the other strand contains no modified bases (deoxyribonucleotides naturally occurring in DNA are not considered modified bases for these purposes). In still other embodiments, eight 2'-OMe modifications are present on one strand of the RNAi agent, while the other strand contains four 2'-OMe modifications.

In yet other embodiments, an RNAi agent used in the described methods and compositions is chemically modified with 2'-F.

In still other embodiments, the modification is selected from 2'-O-methyl (2'-OMe), 2'-O-(2-methoxyethyl) (MOE), and 2'-fluorine.

In other embodiments, the chemical modification is a modification described in paragraphs 0040-0050 of US Patent Application Pub. No. 2011/0195123.

In other embodiments, the chemical modification is a modification to the overhang(s) and/or termini, or to the duplex architecture, as described in paragraphs 0061 and 0062, respectively, of US Patent Application Pub. No. 2011/0195123.

In other embodiments, an RNAi agent used in the described compositions may be conjugated to a molecule. In more specific embodiments, a non-nucleotide molecule is used. In more specific embodiments, the molecule may be cholesterol, a cell-penetrating peptide, spermine, a hydrophobized hyaluronic acid-spermine conjugate (HHSC), or alpha-tocopherol-vitamin E. In certain embodiments, the cholesterol may be conjugated to the 3' end of the sense strand. In other embodiments, the cholesterol may be conjugated to the 5' end of the sense strand. In certain embodiments, in the case of a hairpin-shaped molecule, the cholesterol may be conjugated to the loop. In other embodiments, the non-nucleotide molecule is a molecule described in paragraphs 0051-0060 of US Patent Application Pub. No. 2011/0195123.

In certain embodiments, the RNAi agent is associated, either via covalent attachment or via non-covalent complexation, with a cell-penetrating peptide (CPP), also referred to as a protein transduction domain (PTD). A CPP is a peptide that has the ability to traverse the plasma membrane and facilitate the delivery of a molecular cargo to the cytoplasm. CPP's include HIV-1 Tat (NCBI Gene ID: 155871) or a fragment thereof comprising the sequence YGRKKRRQRRR (SEQ ID No: 3); pAntp (penetratin) and pIs1, which originate from the third helix of homeodomain proteins (Antennapedia (NCBI Gene ID: 40835; Terrone et al., Biochemistry. 2003; 42(47):13787-99) and Isl-1 (NCBI Gene ID: 3670 and Magzoub et al., Biochim Biophys Acta. 2001;1512(1):77-89), respectively); Transportan, a synthetic chimera of galanin and mastoparan (GWTLNSAGYLLGKINLKALAALAKKIL-amide [SEQ ID No: 4]; Pooga et al., FASEB J. 1998 Jan;12(1):67-77), MPG (GALFLGFLGAAGSTMGA [SEQ ID No: 5]); Pep-1 (KETWWETWWTEW SEQ ID No: 6]); and secondary amphipathic peptides based on aromatic tryptophan and arginine residues linked with lysine as spacer ("CADY"), which contain a short peptide sequence of 20 amino acids, with the sequence "Ac-GLWRALWRLLRSLWRLLWRA-cysteamide" (SEQ ID No: 7). CPP's are known to those skilled in the art and are described inter alia in Deshayes et al. (Biochemistry 2004; 43(24):7698-706).

In other embodiments, an RNAi agent used in methods and compositions described herein may be complexed with a cationic polymer, including DOTMA {N-[1-(2,3-dioleyloxy)propyl]-N,N,N-trimethylammonium chloride}, DOTAP (1,2-dioleoyl-3-trimethylammonium-propane)], a polycation including DOGS (dioctadecylamidoglycylspermine) and lipopolyamine RPR120535 or guanidinium groups including BGTC, bis(guanidinium)-tris(2-aminoethyl)amine-cholesterol, a colipid including dioleoyl phosphatidylethanolamine (DOPE), spermine, PEI, a PEI-PLA polymer, N-Acetylgalactosamine (GalNAc), or a lipidic aminoglycoside derivative, including BGTC/DOPE and DC-Chol/DOPE. In other embodiments, the non-nucleotide molecule is a molecule described in paragraphs 0051-0060 of US Patent Application Pub. No. 2011/0195123. In more specific embodiments, particles present in the described methods and compositions are L-PLA particles containing an siRNA-DOTAP Complex, in other embodiments containing an siRNA-DOPE complex, in other embodiments containing an siRNA-PEI complex, and in other embodiments containing an siRNA-spermine complex.

In other embodiments, the particles comprise DOTAP-siRNA conjugates at an n/p ratio between 1 and 5, inclusive. In other embodiments, the particles comprise PEI-siRNA conjugates at an n/p ratio between 1 and 10, inclusive. In still more specific embodiments, the MW of the PEI polymer is between 2000-30,000, inclusive.

In other embodiments, the particles comprise DOTAP-siRNA conjugates at an n/p ratio between 1 and 5, inclusive. In other embodiments, the particles comprise PEI-siRNA conjugates at an n/p ratio between 1 and 10, inclusive. In still more specific embodiments, the MW of the PEI polymer is between 2000-30,000, inclusive.

In other embodiments, the RNAi agent is associated with a CPP mimetic. CPP mimetics are known to those skilled in the art and are described, inter alia, in US Patent Application Pub. No 2008/0139493 (Selwood et al).

In other embodiments, an RNAi agent used in the described methods and compositions is a hairpin-shaped molecule. In another embodiment, the RNAi agent is a double-stranded molecule containing 2 separate strands. In another embodiment, the RNAi agent is selected from the group consisting of a small interfering RNA (siRNA), a short hairpin RNA (shRNA), a Dicer-substrate siRNAs (DsiRNAs), a microRNA, and a non-coding RNA.

### Additional therapeutic agents

In other embodiments, the described DDD comprises more than one drug, for example, an RNAi agent and a chemotherapy agent, and in another example an RNAi agent and a immunotherapy agent. In certain embodiments, one drug is encapsulated in the particles, while the other may not. For example, an RNAi agent may be encapsulated, while a chemotherapy agent is dispersed in the matrix. In other embodiments, an RNAi agent is present both within the particles and in the matrix, and a chemotherapy agent is dispersed in the matrix.

Chemotherapy drugs that may be used include but are not limited to pyrimidine analogues, non-limiting examples of which are 5-azacytidine, 5-aza-2'-deoxycytidine, 5-fluoro-uracil, 5-fluoro-deoxyuridine (floxuridine), and 5-fluorodeoxyuridine monophosphate. In more specific embodiments, the anti-cancer agent is an inhibitor of ribonucleoside-diphosphate reductase large subunit (EC 1.17.4.1), non-limiting examples of which are motexafin gadolinium (CHEBI: 50161); hydroxyurea; gemcitabine (2',2'-difluorodeoxycytidine); elacytarabine (CP-4055; an ara-C-5'elaidic-acid-ester) and CP-4126, (CO 1.01; a gemcitabine-5'elaidic-acid-ester; Adema AD et al, Metabolism and accumulation of the lipophilic deoxynucleoside analogs elacytarabine and CP-4126. Invest New Drugs. 2011 Oct 15), and those described in International Patent Publication No. WO2011/062503. In other embodiments, the anti-cancer agent comprises gemcitabine. In alternative embodiments, the anti-cancer agent is gemcitabine. In yet other embodiments, the anti-cancer agent is an EGFR tyrosine kinase inhibitor. In yet other embodiments, the anti-cancer agent comprises a thymidylate synthase inhibitor. In more specific embodiments, the anti-cancer agent comprises leucovorin (Folinic acid; 2-[[4-[(2-amino-5-formyl-4-oxo-1,6,7,8-tetrahydropteridin-6-yl)methylamino]benzoyl] amino]pentanedioic acid). In yet other embodiments, the anti-cancer agent comprises irinotecan. In yet other embodiments, the anti-cancer agent comprises oxaliplatin. In still other embodiments, the anti-cancer agent comprises FOLFIRIN (5-fluorouracil, leucovorin, and irinotecan in combination). In still other embodiments, the anti-cancer agent is FOLFIRINOX (5-fluorouracil, leucovorin, irinotecan, and oxaliplatin in combination), or any combination of a subset of the four agents in FOLFIRINOX. In yet other embodiments, the anti-cancer agent comprises an EGFR tyrosine kinase inhibitor. In more specific embodiments, the anti-cancer agent is Erlotinib.

In other embodiments, the DDD comprises an immunotherapy agent (Guo et al ("In situ vaccination with CD204 gene-silenced dendritic cell, not unmodified dendritic cell, enhances radiation therapy of prostate cancer." Molecular Cancer Therapeutics, Mol Cancer Ther. 2012 Nov 6. [Epub ahead of print]) and references therein; Clinical Immunotherapy Trials Network www.CITNinfo.com). Non-limiting examples of immunotherapy agents are ipilimumab (Yervoy; Bristol-Myers Squibb), sipuleucel-T (Dendreon Corp, Seattle, WA), IL-7, CP-870,893 (Pfizer), Allovectin-7 (Vical), BiovaxID (Biovest International), IMA901 (Immatics Biotechnologies GmbH), MAGE-A3 (GlaxoSmithKline), Multikine (CEL-SCI Corporation), NeuVax (Galena Biopharma), PROSTVAC (Bavarian Nordic A/S), Rindopepimut (CDX-110) (Celldex Therapeutics), Stimuvax (Oncothyreon and Merck KGaA), Talimogene laherparepvec (Amgen), and TG4010 (Transgene and Novartis). In certain embodiments, the immunotherapy agent does not comprise live cells.

Numerous types of immunotherapeutic agents have been developed, and a number of intratumoral immunotherapies are currently being examined in clinical trials (Cancer Immunotherapy Trial Network www.CITNinfo.org). Agents include T-cell and NK-cell growth factors like IL-15, others that stimulate T cells or activate dendritic cells, so-called immune checkpoint inhibitors like ipilimumab, and others that inhibit or neutralize factors secreted by tumors that suppress the immune system. Recently, two agents have been selected to be tested in CITN-led trials, selected from the 20 identified in the CITN 2007 workshop, IL-15 and a dendritic cell-activating monoclonal antibody called CP-870,893.

Immunotherapy agent interleukin-15 (IL-15) is a recombinant agent that is chemically identical or similar to the endogenous cytokine interleukin-15 (IL-15) with immunomodulating activity. IL-15, secreted by mononuclear phagocytes (and some other cell types) following viral infection, regulates T and natural killer cell activation and proliferation. This cytokine induces activation of transcription activators STAT3, STAT5, and STAT6 via JAK kinase signal transduction pathways in mast cells, T cells, and dendritic epidermal T cells.

CP-870,893 is a fully human monoclonal antibody (mAb) agonist of the cell surface receptor CD40 with potential immunostimulatory and antineoplastic activities. Similar to the CD40 ligand (CD40L or CD154), CD40 agonist monoclonal antibody CP-870,893 binds to CD40 on a variety of immune cell types, triggering the cellular proliferation and activation of antigen-presenting cells (APCs), activating B cells and T cells, and enhancing the immune response; in addition, this agent may activate CD40 present on the surfaces of some solid tumor cells, resulting in apoptosis and decreased tumor growth.

In other embodiments particles leave the DDD and enter the bloodstream. In some embodiments, the particles are associated with a targeting moiety. As an example DDD implanted in the pancreatic tumor release particles of which significant part of them is streamed to the blood and reached the liver. Particles are dissolved at the liver and for example affect metastasis, releasing the same drug, for example the same siRNA designed to halt the tumor growth, for example siRNA anti K-RAS mutated.

### Further embodiments

In other embodiments, the DDD is made of thin polymeric fibers, including electrospun fiber, melt fibers (for example as described in as described in US 2011/0195123 to Shemi)

In some embodiments, the matrix of the DDD is biodegradable. In other embodiments, the DDD is bio-stable, namely highly or totally resistant to degradation *in vivo,* for example DDD made of an elastomer such as silicon.

### Methods of Manufacturing

Also described herein is a method of preparing a millimeter-scale DDD, comprising the steps of: (a) forming small particles comprising a first polymeric material and an RNAi agent; and (b) forming a matrix comprising a second polymeric material and the small particles formed in step (A). As previously described, the first and second polymeric materials are typically biodegradable.

In some embodiments, the aforementioned process comprises the step of combining the small particles of step (a) with the second polymeric material or its monomers, in a solvent in which the small particles are substantially insoluble, but the second polymeric material or its monomers are soluble. "Substantially insoluble" in this regard refers to a loss in particle weight of not larger than 20% after mixing the particles with the solvent.

Those skilled in the art will appreciate in light of the present disclosure that, while ethyl acetate is exemplified herein, a variety of suitable solvents may be used, for example Chloroform, Dichloromethane (DCM or methylene chloride), Tetrahydrofuran (THF), Acetone, Ethyl methyl ketone, and mixtures thereof.

When using ethyl acetate as the solvent, for example, the particles may comprise various polymers. In certain embodiments, when ethyl acetate is used as the solvent, the particles are composed of a polymer that is substantially insoluble in ethyl acetate. Non-limiting examples of such polymers are poly(L-lactide) (L-PLA), PGA (poly(glycolide)), PCL (poly(ε-caprolactone), PLCL (Poly(DL-lactide-co-ε-caprolactone), and Chitosan. In further embodiments, for example when ethyl acetate is used as the solvent and the particles are composed of one of the above materials, the polymeric material in the matrix may be DL-PLA poly(DL-lactide) or PLGA, e.g. L-PLGA or DL-PLGA.

In other embodiments, the particles of the methods and compositions described herein are selected from the following groups: Poly(anhydrides), Poly(ketal), Poly(ortho esters), Poly(acetal), Poly(e-caprolactone), and Poly(a-hydroxy-esters)

Methods of forming the DDD matrices described here typically are performed in room temperature.

In other embodiments the DDD matrix is formed by fiber mats. Fiber can be formed by electro-spinning methods or by other methods, including fiber spinning. The encapsulation of the particles within such a fiber mat in some embodiments is done by spraying the particle during or after mat formation, and/or by dipping.

In other methods the DDD matrix is formed by pressing. In other methods the DDD matrix is formed by RTV (room temperature vulcanization) methods.

Other embodiments provide a matrix or DDD manufactured by one of the methods mentioned herein.

### Drug delivery devices and therapeutic methods

As provided herein, the described millimeter-scale drug delivery devices (DDD) comprising matrices can be manufactured and efficaciously used.

"Millimeter-scale", as used herein, refers to a device whose smallest diameter is at least 0.24 mm (fitting within the inner diameter of a 25-gauge needle). In certain preferred embodiments, each of the dimensions (diameter, in the case of a sphere or cylinder; and height and/or width or length, in the case of a cylinder, box-like structure, cube, or other shape with flat walls) is between 0.3 - 10 mm, inclusive. In more preferred embodiments, each dimension is between 0.5 - 8 mm, inclusive. In more preferred embodiments, each dimension is between 0.6 - 1.3 mm, inclusive.

In more preferred embodiments, the device is a cylinder, having a diameter of 0.1-2.5 mm, inclusive, more preferably 0.2-2.0 mm, more preferably 0.3-1.5 mm, more preferably 0.38-0.98 mm, more preferably 0.48-0.88 mm, more preferably 0.53-0.83 mm, more preferably 0.58-0.78 mm, more preferably 0.63-0.73 mm, more preferably 0.68 mm. In other preferred embodiments, the cylinder has a length of 0.5-3.5 mm, inclusive, more preferably 0.7-3.0 mm, more preferably 0.9-2.5 mm, more preferably 1.0-2.0 mm, more preferably 1.1-1.5, more preferably 1.3 mm. In other embodiments, the cylinder has a diameter of 0.38-0.98 mm, inclusive and a length of 0.5-3.5 mm, inclusive. In more preferred embodiments, the diameter is 0.48-0.88 mm, and the length is 0.7-3.0 mm. In more preferred embodiments, the diameter is 0.53-0.83 mm, and the length is 0.9-2.5 mm. In more preferred embodiments, the diameter is 0.58-0.78 mm, and the length is 1.0-2.0 mm. In more preferred embodiments, the diameter is 0.63-0.73 mm, and the length is 1.1-1.5 mm. In more preferred embodiments, the diameter is 0.68 mm, and the length is 1.3 mm. In still other embodiments, the device is a suitable size for administration with a endoscope and a 19-gauge needle.

In other embodiments, the volume of the device is between 0.1 mm³ and 1000 mm³.

In other embodiments, the DDD is designed with a particular release profile. One relevant parameter is the time point at which 95% of the particles have been released. In some embodiments, the DDD releases 95% of the particles over a time period between 3-24 months inclusive, for example 3, 4, 5, 6, 8, 10, 12, 14, 16, 18, 20, 22, or 24 months. In other embodiments, the time point of release of 95% of the particles is between 3-12 months inclusive, between 2-24 months inclusive, between 2-15 months inclusive, between 3-15 months inclusive, between 3-12 months inclusive, between 3-10 months inclusive, between 4-24 months inclusive. Another relevant parameter is the time point at which 90% of the particles have been released; this may be any of the aforementioned time frames.

Another relevant parameter is the time point at which 95% of the active agent has been released. In some embodiments, the DDD releases 95% of the active agent over a time period between 3-24 months inclusive, for example 3, 4, 5, 6, 8, 10, 12, 14, 16, 18, 20, 22, or 24 months. In other embodiments, the time point of release of 95% of the active agent is between 3-12 months inclusive, between 2-24 months inclusive, between 2-15 months inclusive, between 3-15 months inclusive, between 3-12 months inclusive, between 3-10 months inclusive, between 4-24 months inclusive. Another relevant parameter is the time point at which 90% of the active agent has been released; this may be any of the aforementioned time frames.

Also disclosed is the use of a composition or a drug delivery device (DDD) as specified herein in a method of treating cancer, comprising the step of administering to a subject in need thereof a DDD described herein, thereby treating a cancer.

The subject may be a human patient. The subject may be a veterinary patient. The cancer may be a solid tumor. The cancer may be an inoperable solid tumor. The cancer may be selected from the group consisting of pancreatic cancer, prostate cancer, cervical cancer, Glioblastoma multiforme (GBM), breast cancer, lung cancer, CRC (colorectal) cancer, thyroid cancer, bladder cancer, and esophageal cancer.

In certain embodiments, an siRNA of methods and compositions described herein targets a gene selected from polycomb ring finger oncogene (BMI-1), human telomerase reverse transcriptase (hTERT), interleukin 6 signal transducer (IL6ST) /gp130, CD44, pregnancy-associated plasma protein A (Pappalysin; PAPPA), Neurophilin and tolloid-like 2 (NETO2), Protein tyrosine phosphatase receptor α (PTPRA), Bromodomain containing 4 (BRD4), ErbB3/HER3, PSCA prostate stem cell antigen (PSCA), Enhancer of zeste homolog 2 (EZH2), Transmembrane protease, serine 2 (TMPRSS2)/ Ets Related Gene (ERG) gene fusion (TMPRSS2/ERG), Carbonic anhydrase XII (CA12), MEK4/MAP2K4, p63/KET, Transmembrane and coiled-coil protein 1 (TMCC1), TMCC2, TMCC3, Neurotrimin (NTM), Cluster of Differentiation 70 (CD70), Transmembrane protein 50B (Tmem50b), Claudin-11 (CLDN11), and Neuroplastin (NPTN).

Also disclosed is the use of a composition or a drug delivery device (DDD) of the present disclosure further comprises the step of administering an anti-cancer agent incorporated within the DDD, or, in other embodiments, in addition to the DDD, to the patient. Suitable anti-cancer agents include but are not limited to those mentioned herein.

The anti-cancer agent may be administered to the patient after administration of the DDD. The anti-cancer agent may be administered to the patient up to 10 days after administration of the DDD. Alternatively, the anti-cancer agent is administered to the patient simultaneously with administration of the DDD. The anti-cancer agent may be administered to the patient before administration of the DDD. In yet other embodiments, the DDD is administered during ongoing administration of the anti-cancer agent. The anti-cancer agent may be administered to the patient intratumorally, by a method such as injection and controlled release, or including in other embodiments administration from the same DDD.

In yet other embodiments, the amount of said RNAi agent in all the DDD's administered as a batch (a single dose) is at least 4 µg, for example at least 5 µg, at least 6 µg, at least 7 µg, at least 8 µg, at least 10 µg, at least 12 µg, or at least 15 µg,. In still other embodiments, the amount of RNAi agent present per dose is between 2-10 µg, inclusive, for example 2, 3, 4, 5, 6, 7, 8, 9, or 10 µg.

### Animal and human testing

In animal models, tumor progress may be monitored by any method known in the art. One method is by removing and weighing the tumor. This may be done, for example, by weighing slices after histology slice preparation.

The presence and amount of therapeutic siRNA in tissue samples may be determined by any method known in the art, for example by a method described herein. RNA quantity may be assessed, for example, by Nanodrop, and RNA quality by gel electrophoresis. RNA quantity may also be assessed by PCR, e.g. real-time PCR, Northern blot, HPLC, MSLC (Membrane surface liquid culture), or *in situ* hybridization.

The presence of particles in tissue samples may be determined by any method known in the art, for example by the methods described herein.

Excised tumor tissue from human or animal studies may be preserved by any method known in the art, for example by freezing in liquid nitrogen (for subsequent studies requiring live cells), or may be fixed, for example in paraformaldehyde solution. Characterization of tissue samples may include various methods known in the art, including but not limited to hematoxylin and eosin staining, immunohistochemistry staining, and measuring levels of gene products, such as the genes targeted by the therapeutic siRNA, in some cases in the presence of an internal control.

In other cases, the effects of devices described herein on the excised tissue sample may be studied, for example in an experimental animal or in culture.

Wherever alternatives for single separable features such as, for example, a first polymeric material (particles), a second polymeric material (matrix), an RNAi agent modification, an RNAi agent conjugation, a particle zeta potential, or a size distribution are laid out herein as "embodiments", it is to be understood that such alternatives may be combined freely to form discrete embodiments of the invention disclosed herein.

The invention is further illustrated by the following examples and the figures, from which further embodiments and advantages can be drawn. These examples are meant to illustrate the invention but not to limit its scope.

### Experimental Details Section

### Reference Example 1: Preparation of siRNA-loaded PLA particles by w/o/w emulsion

1. The aqueous phase, 245 µg siRNA in 200 µl PBS/TE buffer, was slowly (over 30s) added to the organic phase, PLA solution (30 mg in 1 ml Dichloromethane [DCM]), and vortexed for 1 min after each addition.
2. The tube was placed on an ice bath for 5 min.
3. The sample was sonicated for 30s.
4. The emulsion was added in two portions to 6 ml PVA 2.5% and vortexed, placed on an ice bath for 5 min, and sonicated for 2 min.
5. The emulsion was stirred for a few hours to allow the evaporation of DCM and formation of particles.
   Optional: (Stir one hour in a desiccator)
6. Particles were centrifuged at 13,000 rpm for 20 min at 4°C, washed twice with water, centrifuged at 13,000 rpm for 20 min at 4°C, resuspended in 5 ml of water and sonicated for 30s in an ice bath.
7. Particles were centrifuged at 1,000 rpm for 10 min at 4°C, then lyophilized for 2 days.

### Reference Example 2: PLA particles containing siRNA-PEI complexes

Referring to Example 1, pre-formed complexes of siRNA and PEI were initially prepared by adding PEI (100µg/ml) to 0.2 ml of an aqueous solution containing 245 µg siRNA and incubating for 30 min at room temperature. This mixture was emulsified in 1 ml of chloroform containing 30mg of polymer as described above.

### Reference Example 3: Preparation of siRNA-complexes with DOTAP

Complexes: For 0.6 mg siRNA), 2.7 mg DOTAP.

### Protocol:

Incubate 2 tubes for each reaction at RT (room temperature) for 5 min:
∘ 0.5 ml double-distilled RNAse-free water + 0.6 mg siRNA 2.7 mg DOTAP reagent)

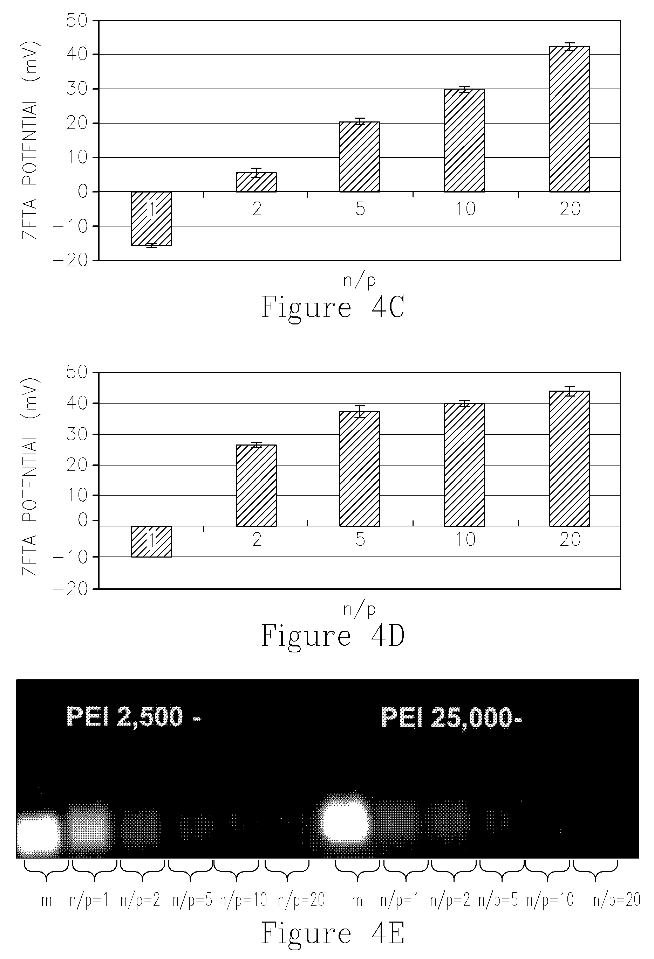

Combine the two tubes and incubate at RT for 30 min. Analyze by gel electophoresis (Figure 4).

### Reference Example 4: Preparation of particles containing siRNA and PLA-PEI copolymer

A similar procedure is used as described in Example 1, except that the polymer solution in DCM also contained varying concentrations of PEI (5-5000µg PEI/30mg PLA).

### Example 5: Preparation and characterization of a PLGA matrix containing PLA particles

Synthesis of fluorescent particles: PLA fluorescent particles were synthesized by nanoprecipitation. The polymer (30 mg L-PLA) and coumarin 6 (1 mg) were dissolved in chloroform (2 ml) fluorophore:PLA concentration of 4% w/w, and this solution was added dropwise to an ethanol solution (50 ml) under magnetic stirring over 2 h. The particles were centrifuged at 13,000 rpm for 15 min at 40°C, washed three times in water and resuspended in water.

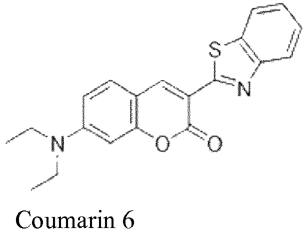

siG12D/DOTAP-loaded PLA particles were also synthesized by nanoprecipitation. First, 2.5 mg siG12D, having the sequence GUUGGAGCUGAUGGCGUAGdTdT (sense strand; SEQ ID No: 1), and CUACGCCAUCAGCUCCAACdTdT (antisense strand; SEQ ID No: 2) was complexed with DOTAP (N-[1-(2,3-Dioleoyloxy)propyl]-N,N,N-trimethylammonium) (11 mg; nitrogen/phosphate (n/p) ratio of 2) in water for 30 min and transferred to chloroform. 30 mg PLA was added to the complex and vortexed for 2 min. The siG12D/DOTAP/PLA solution was added dropwise to a 50-ml ethanol solution under magnetic stirring for 2 h. The resulting particles were centrifuged at 13,000 rpm for 15 min at 40°C, washed three times, resuspended in water, and freeze-dried for 48h.

### Characterization of particles:

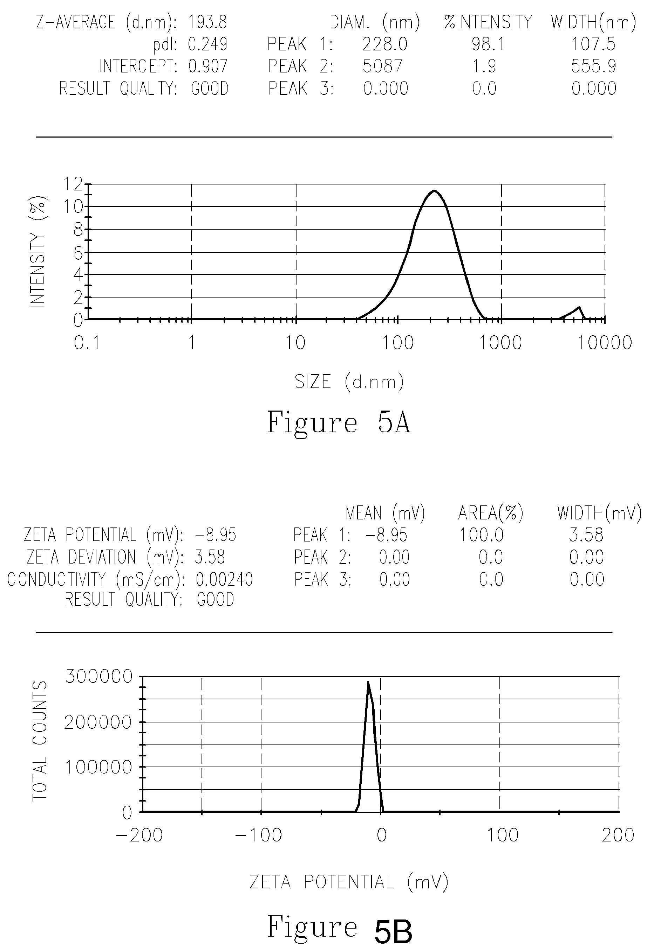

The size of the particles was measured. Most particles were between 80-500 nm (nanometers) in diameter (Figure 5A). Additionally, the zeta potential of the particles was measured, using Zetasizer (Malvern Instruments Ltd, United Kingdom). The zeta potential of most particles was between about -20 and 0 millivolts (mV) (Figure 5B). The PDI (Poly Dispersity Index, a measure of relative variance in the particle size distribution) of the particles was 0.181.

### Preparation of particle-containing matrix

PLA particles (20 mg), mannitol (10 mg), sodium bicarbonate (0.25 mg), and PLGA (100 mg) were mixed, and 400 µl of Ethyl Acetate was added. The suspension was injected into a 19G Teflon® tube, and the solvent was evaporated to obtain the matrix.

### Example 6: Characterization of PLA particles after a three-week incubation of the DDD in PBS

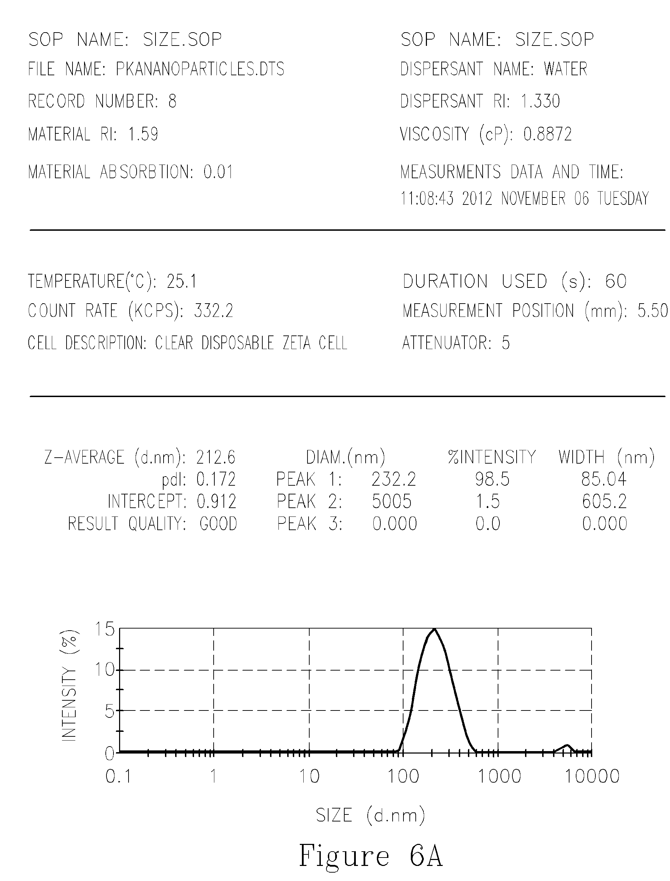

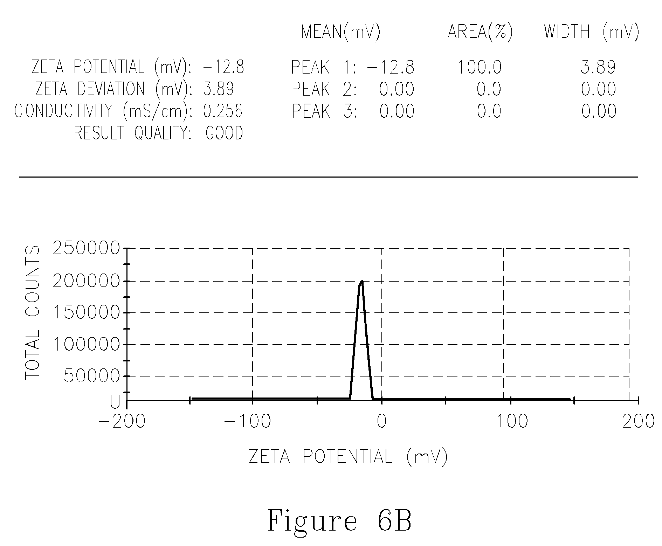

After storage in PBS at 37°C for three weeks, the matrix of Example 5 was dissolved in ethyl acetate, and the size and zeta potential of the particles was determined. The majority of the particles exhibited the same characteristics as prior to their incorporation into the particles (Figure 6A-B). This demonstrated that particles retained their structure within the matrix.

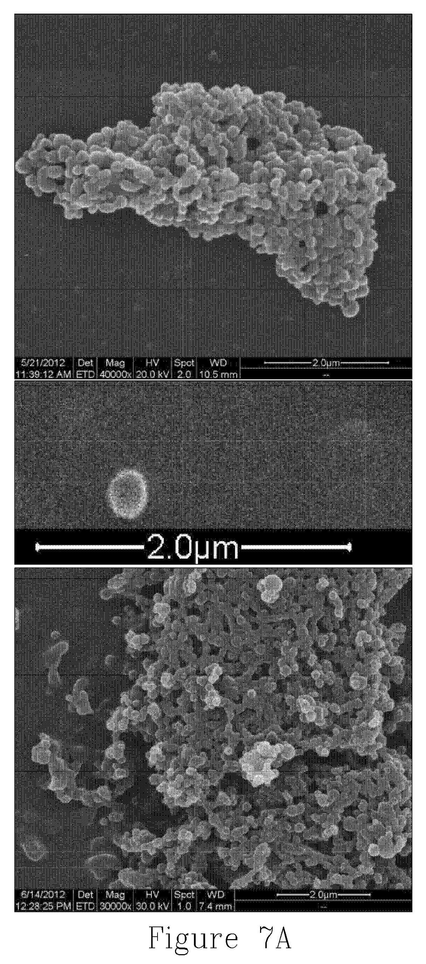

This finding was confirmed by scanning and transmission electron microscopy of the released particles, showing their structural integrity (Figure 7).

### Additional studies

L-PLA nanoparticles were synthesized by nanoprecipitation, and their size was measured. Particle-containing DDD matrices was constructed. One matrix was incubated in PBS at 37°C for 7 days, after which the released nanoparticles were collected and measured.

A second matrix was incubated at 4°C for 3 weeks, after which the matrix was dissolved and the nanoparticles were collected and measured.

A third matrix was incubated in PBS 37°C for 3 weeks and treated as described above.

The size of the nanoparticles remained rather stable (~200 nm) even after 3 weeks, with a maximal change in diameter of 33%, and a 52% increase in PDI, in the case of particles extracted from DDD after 21 days at 37°C in PBS.

**Table 1. Characteristics of particles before and after storage of the matrix in PBS.**

| ***L-PLA Nanoparticles*** | ***Size (d, nm)*** | ***PDI*** |
|---|---|---|
| Nanoparticles (t=o) | 194 ± 5 | 0.249 |
| Nanoparticles released from DDD (7 days, 37°C in PBS) | 212.6 ± 10 | 0.242 |
| Nanoparticles extracted from DDD (21 days, 4°C) | 221.3 ± 9 | 0.262 |
| Nanoparticles extracted from DDD (21 days, 37°C in PBS) | 291.3 ± 25 | 0.312 |

### Example 7: Time course of particle release from matrix into PBS

The matrix of Example 5 was incubated in PBS (phosphate-buffered saline) at 37°C, in a humidified incubator, and particles were collected by centrifugation and lyophilized. There was a gradual release of about 25% of the particles over the first 20 days (Figure 8).

### Example 8: Time course of diffusion of particles from the matrix into gelatin

Gelatin from porcine skin (Sigma cat #G1890)] The coumarin 6-containing matrix of Example 5 was incubated in 10% gelatin, and fluorescence in the matrix and surrounding region was observed under a microscope at 4 hours and 1, 4, 6, and 7 days. The particles diffused steadily into the gelatin (Figure 9A). The diffusion distance was proportional to the square root of the elapsed time (Figure 9B-C). Naked siRNA diffused significantly faster than the nanoparticles (Figure 10).

### Example 9: Preparation of drug delivery devices

Drug delivery devices (DDD's) are prepared from a particle-containing matrix, for example prepared as described in Example 5, by pouring the particles/polymer suspension to a desired thickness and excising pieces/chunks of matrix in the desired shape and thickness.

### Example 10: Characterization of particles with various contents

Particles containing additional molecules were produced and characterized. The results are shown in Table 2 below.

**Table 2. Characteristics of particles with various contents.**

| **Particle contents** | **Zeta potential** | **Size of particles** |
|---|---|---|
| Methylene blue: | -28 mV | 187 nm |
| MW 319 g/mol | | PDI: 0.286 |
| 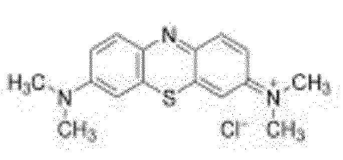 | | |
| Coumarin 6 | -30 mV | 132 nm |
| MW: 350 g/mol | | PDI: 0.181 |
| siG12D-DOTAP | -10 mV | 80 nm |
| | | PDI: 0.131 |
| Methyl Orange | -50 mV | 125 nm |
| 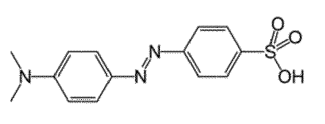 | | PDI: 0.262 |
| (none) | -3 mV | 200 nm |

### Reference Example 11: Characterization of other types of particles

Several types of particles have been tested, including PLA nanoparticles containing an siG12D-DOTAP Complex, PLA nanoparticles containing an siG12D-DOPE complex, PLA nanoparticles containing an siG12D-PEI complex, and PLA nanoparticles containing siG12D-spermine complex.

A pre-formed complex of siRNA and DOTA/ PEI/ DOPE/ spermine was initially prepared by adding DOTA/ PEI/ DOPE/ spermine to an aqueous solution containing siRNA and incubating for 30 min at room temperature. The complex was transferred to chloroform.

PLA (30 mg) was added to the complex and vortexed for 2 min. The siG12D complex /PLA solution was added dropwise to a 50-ml ethanol solution under magnetic stirring for 2 h. The resulting particles were centrifuged at 13,000 rpm for 15 min at 4°C, washed three times, re-suspended in water, and freeze-dried for 48 h.

In other experiments, other types of particles (for example particles having varying polymer compositions and payloads, and synthesized using alternative protocols) are produced and characterized for their size, zeta potential, and PDI, using methods known in the art. Possible payloads include RNAi agents and other drugs. The diffusion characteristics in PBS and gelatin are measured.

### Reference Example 12: Characterization of particle release in tumor tissue

Tumors are implanted into mice by methods known in the art, for example as described in Example 12 of WO2010/001325 to Shemi. Particle-containing DDD containing a fluorophore are implanted in the vicinity of the tumor, and release of particles into the surrounding tissue is monitored by imaging.

### Reference Example 13: Characterization of siRNA release in tumor tissue

Tumors are implanted into mice by methods known in the art. Particle-containing DDD containing siRNA complexed to a material that undergoes FRET (fluorescence-resonance energy transfer), for example DOTAP, are implanted in the vicinity of the tumor. siRNA release is monitored by measuring FRET.

### Reference Example 14: Animal testing of particle-containing DDD anti-tumor activity

Using a mouse xenograft tumor model or other suitable model, one or more particle-containing DDDs are implanted in the tumor. The DDD's contain an anti-mutated K-RAS siRNA (hereinafter siG12D), or another suitable siRNA, in both the matrix and the particles. Tumor progress and/or the amount of therapeutic siRNA in the tissue are monitored. In some experiments, an siRNA-containing DDD without particles, for example as described in in US 2011/0195123 (Shemi), is used as a control.

### Reference Example 15: Clinical trials of particle-containing DDD

One or more DDD's, for example such as the DDD's described in the previous Example, are implanted in a solid tumor. One example is a tumor of a patient with a resectable pancreatic tumor. In some embodiments, the DDD's are implanted prior to the tumor removal operation (usually called a Whipple procedure or Whipple surgery). The tumor tissue adjacent and non-adjacent to the DDD site are characterized for morphology, therapeutic siRNA content, and/or expression of the gene targeted by the therapeutic siRNA.

It will be apparent that the precise details of the methods and compositions described herein may be varied or modified without departing from the spirit of the described invention.

## Claims

1. An implant, comprising:
A. particles between 4 nm - 5 µm, inclusive, comprising a first polymeric material, wherein said first polymeric material is selected from the group consisting of: polylactic acid (PLA), polyglycolic acid (PGA); poly caprolactone (PCL); and PLCL (Poly(DL-lactide-co-ε-caprolactone);
B. a matrix comprising a second polymeric material non-identical to said first polymeric material; and
C. an RNAi (RNA interference) agent,
and wherein said particles are incorporated within said matrix, and some or all of said RNAi agent is incorporated within said particles.

2. The implant of claim 1, wherein said matrix is biodegradable.

3. The implant of any one of claims 1 or 2, where said second polymeric material comprises PLGA
a. with a LA: GA ratio between 25:75 and 75:25 or
b. with a LA:GA ratio between 75:25 and 95:5.

4. The implant of any one of the previous claims, wherein said first polymeric material shares at least one building block with the polymer in said second polymeric material.

5. The implant of any one of the previous claims, wherein the zeta potential of said particles at pH=6.75 is between -30mV and +10mV, inclusive.

6. The implant of any one of the previous claims, wherein less than 50% by weight of said RNAi agent in said implant is contained in said particles, and the remainder is contained in said matrix.

7. The implant of any one of the previous claims, wherein, said particles have a size distribution selected from the group consisting of:
- a single size peak in the range between 20 to 500 nm, inclusive; and
- a biphasic size distribution, having a first size peak larger than 200 nm, and a second size peak smaller than 120 nm.

8. The implant of any one of the previous claims, wherein said RNAi agent is chemically modified with a modification selected from the group consisting of 2'-O-methyl (2'-OMe), 2'-O-(2-methoxyethyl) (MOE), and 2'-fluorine.

9. The implant of any one of the previous claims, wherein said RNAi agent is conjugated to a molecule selected from the group consisting of a cholesterol moiety, spermine, a hydrophobized hyaluronic acid-spermine conjugate (HHSC), alpha-tocopherol-vitamin E, or a cell penetrating peptide; or is complexed with a cationic molecule.

10. The implant of claim 1, wherein the implant is of millimeter-scale, and the diameter of said implant is greater by at least a factor of 1 x 10³ than the mean diameter of said particles, and the volume of said implant is larger by at least by a factor of 1 x 10⁹ than the mean volume of said particles.

11. The implant of any of the previous claims, wherein the implant is of millimeter-scale, further comprising an additional therapeutic agent selected from the group consisting of an additional RNAi agent, an immunotherapy agent, and a chemotherapy drug.

12. A method of preparing an implant, comprising the steps of:
A. forming particles between 4 nm - 5 µm, inclusive, comprising a first polymeric material selected from the group consisting of poly(L-lactide) (L-PLA), PGA (poly(glycolide)), PCL (poly(ε-caprolactone), and PLCL (Poly(DL-lactide-co-ε-caprolactone);
and an RNAi agent; and
B. forming an implant comprising a second polymeric material and the particles formed in step (A),
wherein step B utilizes a solvent in which said particles are substantially insoluble.

## Patentansprüche

1. Ein Implantat, umfassend:
A. Partikel zwischen 4 nm bis 5 µm, einschließlich, umfassend ein erstes Polymermaterial, wobei besagtes erstes Polymermaterial aus der Gruppe bestehend aus Polymilchsäure (PLA), Polyhydroxyessigsäure (PGA), Polycaprolacton (PCL) und PLCL (Poly(DL-lactid-co-ε-caprolacton) ausgewählt ist;
B. eine Matrix umfassend ein zweites Polymermaterial, welches nicht identisch zu besagtem ersten Polymermaterial ist, und
C. ein RNAi (RNA Interferenz)-Wirkstoff,
und wobei besagte Partikel in besagter Matrix eingebaut sind, und einiger oder aller besagter RNAi-Wirkstoff in besagten Partikeln eingebaut ist.

2. Das Implantat gemäß Anspruch 1, wobei besagte Matrix biologisch abbaubar ist.

3. Das Implantat gemäß einem der Ansprüche 1 bis 2, wobei besagtes zweites Polymermaterial PLGA
a. mit einem LA: GA Verhältnis zwischen 25:75 und 75:25 oder
b. mit einem LA:GA Verhältnis zwischen 75:25 und 95:5.
umfasst.

4. Das Implantat gemäß einem der vorstehenden Ansprüche, wobei besagtes erstes Polymermaterial mindestens eine Baueinheit mit dem Polymer besagten zweiten Polymermaterials gemein hat.

5. Das Implantat gemäß einem der vorstehenden Ansprüche, wobei das Zetapotential besagter Partikel bei pH=6.75 zwischen -30mV und +10mV einschließlich liegt.

6. Das Implantat gemäß einem der vorstehenden Ansprüche, wobei weniger als 50 Gewichtsprozent des besagten RNAi-Wirkstoffes des besagten Implantats in besagten Partikeln enthalten ist, und der Rest in besagter Matrix enthalten ist.

7. Das Implantat gemäß einem der vorstehenden Ansprüche, wobei besagte Partikel eine Größenverteilung ausgewählt aus der Gruppe bestehend aus:
- einem einzigen Maximum in dem Bereich von 20 bis 500 nm einschließlich und
- einer Zweiphasen-Größenverteilung mit einem ersten Größenmaximum größer als 200 nm und einem zweiten Größenmaximum kleiner als 120 nm
haben.

8. Das Implantat gemäß einem der vorstehenden Ansprüche, wobei besagter RNAi-Wirkstoff durch eine Modifikation ausgewählt aus der Gruppe bestehend aus 2'-O-Methyl (2'-OMe), 2'-O-(2-methoxyethyl) (MOE) und 2'-Fluorid chemisch verändert ist.

9. Das Implantat gemäß einem der vorstehenden Ansprüche, wobei besagter RNAi-Wirkstoff an ein Molekül ausgewählt aus der Gruppe bestehend aus einem Cholesterinrest, Spermin, ein hydrophobisiertes Hyaluronsäure-Sperminkonjugat (HHSC), alpha-Tocopherol-Vitamin E, ein Zell-penetrierendes Peptid oder ein kationisches Molekül konjugiert ist.

10. Das Implantat gemäß Anspruch 1, wobei das Implantat von einer Größe im Millimeter-Bereich ist, und der Durchmesser besagten Implantats um einen Faktor von mindestens 1 x 10³ größer ist als der mittlere Durchmesser besagter Partikel, und das Volumen besagten Implantats um mindestens einen Faktor 1 x 10⁹ größer ist als das mittlere Volumen besagter Partikel.

11. Das Implantat gemäß einem der vorstehenden Ansprüche, wobei das Implantat von einer Größe im Millimeter-Bereich ist, weiter umfassend einen zusätzlichen therapeutischen Wirkstoff ausgewählt aus der Gruppe bestehend aus einem zusätzlichen RNAi-Wirkstoff, einem Immunotherapie-Wirkstoff und einem chemotherapeutischen Arzneistoff.

12. Ein Verfahren zur Herstellung eines Implantats, umfassend die Schritte:
A. Herstellung von Partikeln zwischen 4 nm bis 5 µm einschließlich, umfassend ein erstes Polymermaterial ausgewählt aus der Gruppe bestehend aus Poly(L-Milchsäure) (L-PLA), PGA (Polyhydroxyessigsäure), PCL (Poly(ε-caprolacton), und PLCL (Poly(DL-lactid-co-ε-caprolacton)
und einem RNAi-Wirstoff, und
B. Herstellung eines Implantats umfassend ein zweites Polymermaterial und die in Schritt A hergestellten Partikel,
wobei Schritt B ein Lösungsmittel verwendet, in welchem die besagten Partikel im Wesentlichen unlöslich sind.

## Revendications

1. Implant, comprenant :
A. des particules entre 4 nm et 5 µm inclus, comprenant un premier matériau polymère, dans lequel ledit premier matériau polymère est choisi dans le groupe comprenant : l'acide polylactique (PLA), l'acide polyglycolique (PGA) ; la polycaprolactone (PCL) ; et la PLCL (poly(DL-lactide-co-s-caprolactone) ;
B. une matrice comprenant un second matériau polymère non identique audit premier matériau polymère ; et
C. un agent RNAi (interférence par ARN),
et dans lequel lesdites particules sont incorporées dans ladite matrice, et une certaine quantité ou la totalité dudit agent RNAi est incorporée dans lesdites particules.

2. Implant selon la revendication 1, dans lequel ladite matrice est biodégradable.

3. Implant selon l'une quelconque des revendications 1 ou 2, dans lequel ledit second matériau polymère comprend du PLGA
a. à un rapport LA:GA compris entre 25:75 et 75:25 ou
b. à un rapport LA:GA compris entre 75:25 et 95:5.

4. Implant selon l'une quelconque des revendications précédentes, dans lequel ledit premier matériau polymère partage au moins un synthon avec le polymère dans ledit second matériau polymère.

5. Implant selon l'une quelconque des revendications précédentes, dans lequel le potentiel zéta desdites particules à pH = 6,75 est entre -30 mV et +10 mV inclus.

6. Implant selon l'une quelconque des revendications précédentes, dans lequel moins de 50 % en poids dudit agent RNAi dans ledit implant sont contenus dans lesdites particules, et le reste est contenu dans ladite matrice.

7. Implant selon l'une quelconque des revendications précédentes, dans lequel, lesdites particules ont une répartition granulométrique choisie dans le groupe comprenant :
- une seule taille maximale dans la plage comprise entre 20 et 500 nm inclus ; et
- une répartition granulométrique biphasique, ayant une première taille maximale plus grande que 200 nm, et une seconde taille maximale plus petite que 120 nm.

8. Implant selon l'une quelconque des revendications précédentes, dans lequel ledit agent RNAi est chimiquement modifié par une modification choisie dans le groupe comprenant le 2'-O-méthyle (2'-OMe), le 2'-O-(2-méthoxyéthyle) (MOE), et le 2'-fluor.

9. Implant selon l'une quelconque des revendications précédentes, dans lequel ledit agent RNAi est conjugué à une molécule choisie dans le groupe comprenant un fragment cholestérol, une spermine, un conjugué hydrophobisé d'acide hyaluronique-spermine (HHSC), un alpha-tocophérol-vitamine E, ou un peptide de pénétration cellulaire ; ou est complexé avec une molécule cationique.

10. Implant selon la revendication 1, dans lequel l'implant est à l'échelle du millimètre, et le diamètre dudit implant est plus grand d'au moins un facteur de 1 x 10³ que le diamètre moyen desdites particules, et le volume dudit implant est plus grand d'au moins un facteur de 1 x 10⁹ que le volume moyen desdites particules.

11. Implant selon l'une quelconque des revendications précédentes, dans lequel l'implant est à l'échelle du millimètre, comprenant en outre un agent thérapeutique supplémentaire choisi dans le groupe comprenant un agent RNAi supplémentaire, un agent d'immunothérapie, et un médicament de chimiothérapie.

12. Procédé de préparation d'un implant, comprenant les étapes suivantes :
A. formation de particules entre 4 nm et 5 µm inclus, comprenant un premier matériau polymère choisi dans le groupe comprenant le poly(L-lactide) (L-PLA), le PGA (poly(glycolide)), la PCL (poly(ε-caprolactone), et la PLCL (poly(DL-lactide-co-ε-caprolactone) ;
et un agent RNAi ; et
B. formation d'un implant comprenant un second matériau polymère et les particules formées dans l'étape (A),
dans lequel l'étape B utilise un solvant dans lequel lesdites particules sont sensiblement insolubles.


### SEQUENCE LISTING

<110> Silenseed Ltd.
<120> COMPOSITIONS FOR DRUG DELIVERY AND METHODS OF MANUFACTURING AND USING SAME
<130> SILE 2142/2.3
<150> 61/629,136
   <151> 2011-11-14
<160> 7
<170> PatentIn version 3.5
<210> 1
   <211> 21
   <212> DNA
   <213> Artificial Sequence
<220>
   <223> synthetic construct - siRNA sense strand
<220>
   <221> RNA
   <222> (1)..(19)
<220>
   <221> DNA
   <222> (20)..(21)
<400> 1
   guuggagcug auggcguagt t 21
<210> 2
   <211> 21
   <212> DNA
   <213> Artificial Sequence
<220>
   <223> synthetic construct - siRNA antisense strand
<220>
   <221> RNA
   <222> (1)..(19)
<220>
   <221> DNA
   <222> (20)..(21)
<400> 2
   cuacgccauc agcuccaact t 21
<210> 3
   <211> 11
   <212> PRT
   <213> Human immunodeficiency virus type 1
<400> 3
   Tyr Gly Arg Lys Lys Arg Arg Gln Arg Arg Arg
   1               5                   10
<210> 4
   <211> 27
   <212> PRT
   <213> Artificial Sequence
<220>
   <223> synthetic chimera of galanin and mastoparan
<220>
   <221> amidation
   <222> (27) .. (27)
<400> 4
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 5
   <211> 17
   <212> PRT
   <213> Artificial Sequence
<220>
   <223> synthetic construct
<400> 5
   Gly Ala Leu Phe Leu Gly Phe Leu Gly Ala Ala Gly Ser Thr Met Gly
   1               5                   10                  15
   Ala
<210> 6
   <211> 12
   <212> PRT
   <213> Artificial Sequence
<220>
   <223> synthetic construct
<400> 6
   Lys Glu Thr Trp Trp Glu Thr Trp Trp Thr Glu Trp
   1               5                   10
<210> 7
   <211> 20
   <212> PRT
   <213> Artificial Sequence
<220>
   <223> synthetic construct
<220>
   <221> acylated
   <222> (1) .. (1)
<220>
   <221> cysteamide
   <222> (20) .. (20)
<400> 7
   Gly Leu Trp Arg Ala Leu Trp Arg Leu Leu Arg Ser Leu Trp Arg Leu
   1               5                   10                  15
   Leu Trp Arg Ala
               20